# EUROPEAN PATENT APPLICATION

(11) **EP 3 858 155 A1**
(43) Date of publication of application: **04.08.2021**
(21) Application number: 20154587.8
(22) Date of filing: 30.01.2020
(51) Int. Cl.: A23L 27/30, A23L 27/00, A23L 2/60, A23F 3/16, A23G 3/50, A23G 4/06

(54) **A SWEETENER OR SWEETNESS ENHANCER COMPOSITION**

(71) Applicant: Analyticon Discovery GmbH, 14473 Potsdam (DE); BRAIN AG, 64673 Zwingenberg (DE)
(72) Inventor: Krohn, Michael, 64653 Lorsch (DE); Riedel, Katja, 64625 Bensheim (DE); Kluge, Grit, 14959 Trebbin (DE); Hetterling, Gregor, 12057 Berlin (DE); Tsichrintzi, Fotini, 10707 Berlin (DE); Siems, Karsten, 14552 Michendorf (DE); Descamps, Nicolas, 59262 Sainghin-en-Mélantois (FR); Druon, Amandine, 59800 Lille (FR)
(74) Representative: Daniels, Stefanie Lisa

(57) **Abstract**

Suggested is a broad variety of compounds extracted from natural sources as sweeteners and sweetness enhancers, particular for rebaudiosides.

## Description

### AREA OF INVENTION

The invention relates to novel natural products, which are obtainable by the extraction of plants or microorganisms and its physiologically acceptable salts, which are useful as a sweetener or sweetener enhancer in preparations and compositions, especially oral edible compositions.

### BACKGROUND OF THE INVENTION

Sweetness is one of the primary taste and cravings of both animals and humans. The universal use of naturally occurring and synthetic sweeteners to satisfy this natural craving has not been met without its accompanying physiological disadvantages, e.g. obesity, nutritional imbalance and dental decay. To overcome these unwanted disadvantages considerable research efforts and expenditures have been made to develop alternative compounds, e.g. as substitute for the naturally occurring sweeteners or synthetic sweeteners, which have no food value and are free of caloric input. While these artificial sweeteners enjoyed a wide use, and fulfilled the requirements of a sweet taste with no food value, and could be used without providing calories or damaging teeth, they were frequently found to possess inherent disadvantages that prevented their use for their intended purpose, e.g. because of their toxicity (p-ethoxyphenylurea) or chromosome damage and bladder trouble (sodium cyclamate). Thus, these sweeteners could not be safely recommended for use as a sweetener and are apparently unacceptable for consumption. Saccharin compounds are also commonly used as artificial sweeteners, since cyclamates have been come under governmental restrictions. Although saccharin compounds possess sweetness characteristics, they are undesirable as the sole sweetening agent in most food and beverage compositions because of the lingering bitter aftertaste perceived by most users. While saccharin and the cyclamates have been in common use as artificial sweetening agents for a number of years, there has been more recently discovered a series of new artificial sweeteners.

### RELEVANT PRIOR ART

For example US 3,087,821 (GENTILI ET AL) teaches the use of various dihydrochalcones having sugar substituents (dihydrochalcones glycosides) as sweetening agents. All sweet dihydrochalkones have a licorice like aftertaste and linger in the mouth for some time.

*Siraitia grosvenori* (Luo han guo), a member of the Cucurbitaceae family, is a plant native to some regions of southern Asia and China. The sweet taste of fruits of luo han guo mainly comes from triterpene glycosides generally known as mogrosides. There are a number of mogrosides identified in luo han guo but generally mogroside V (CAS No: 88901-36-4) has the highest concentration compared to others (Table 1). Mogrol glycosides have the same core molecule-mogrol or oxo-mogrol and differ from each other by number and type of glycosidic residues bonded to mogrol or oxo-mogrol molecules [see US 2012/0059071**,** Kasai et al. AGRIC BIOL CHEM (1989), p 3347-3349**;** Matsumoto et. al. CHEM PHARM BULL (1990) p 2030-2032.] Several mogrosides taste very sweet, often >100x sweeter than sucrose (EP 3099186 A2**,** ANALYTICON DISCOVERY), including the major triterpene glycoside of Siraitia grosvenori, mogroside V, and its isomer iso-mogroside V (US 2011 0027413), but all of them have a certain bitter aftertaste. Recently, Senomyx (WO 2017/075257) and PureCircle (WO 2018/089469) disclosed further sweet tasting mogrosides.

Leaves of *Stevia rebaudiana* are well known for its sweet taste due to its content of sweet diterpene glycosides. One of the major sweet compounds from stevia, Rebaudioside A, is approved as natural sweetener in US (since 2008) und EU (since 2011). Apart from its sweet taste, all sweeteners from stevia have a slower onset and longer duration than that of sugar, and a bitter or licorice-like aftertaste at high concentrations [Lemus-Mondaca et al. FOOD CHEM 132 (2012) p 1121-1132**].**

Another example is EP 2529633 A1 (SYMRISE), which relates to triterpenes and triterpene glycosides and/or physiologically acceptable salts thereof. The triterpenes are, preferably naturally occurring triterpenes and triterpene glycosides from *Mycetia balansae,* for generating a sweet impression in an orally consumable formulation or for reinforcing the sweet impression of an orally consumable formulation. These triterpene-glycosides differ in structure from the triterpenes claimed in the present invention.

European patent applications EP 3108754 A1 und EP 3111780 A1 (ANALYTICON) disclose extracts from *Pterocarya* and specific stilbenes as sweeteners or sweetness enhancers.

*Gynostemma pentaphyllum* is traditionally used as a natural sweetening agent in China (International Journal of Research in Pharmaceutical and Biomedical Sciences Vol. 2(4) Oct - Dec 2011, 1483 - 1502). At least 85 triterpene glycosides were isolated from G. pentaphyllum, but only Gypenoside XX is known for its sweet taste (KINGHORN et al., Terpenoid Glycoside Sweeteners in Natural Occurring Glycosides: Chemistry, Distribution and Biological Properties. John Wiley and Sons. Chichester, UK, 1999, p. 399-429.)

Some natural products enhance the sweet taste of sucrose or of high intensity sweeteners although they do not show an intrinsic sweetness. The flavonoid hesperetin enhances the sweetness of sucrose (5% sucrose in water) by 41% at a concentration of 100 pm (EP 2 368 442**,** Symrise). Chromocell describes natural and synthetic 3-hydroxy-flavanols as enhancer of the sweet taste of many high intensity sweeteners including Rebaudioside M **(**WO 2014/153000**).** In WO 2014/186250 PepsiCo discloses the combination of Rebaudioside M with sugar or high intensity sweeteners, e.g. other steviol glycosides or mogrosides to improve the sweet taste. PepsiCo did not describe a combination of a compound without intrinsic sweet taste acting as a sweet taste enhancer of Rebaudioside M.

A more general review of sweeteners and sweetness enhancers can be found in FOOD SCI. TECHNOL. 38(2): 181-187 (2018**).**

### OBJECT OF THE INVENTION

Accordingly, it is a primary object of the present invention to provide novel sweetener compounds and its physiologically acceptable salts, which have a positive sweet benefit in food and oral compositions. In particular, the object was to provide sweetener compounds which are capable to provide sweetness to consumable compositions in a way, that the balance between the degree of sweetness and the amount which has to be administered to obtain a sweet effect is comparable low, to overcome the aforesaid disadvantages associated with the prior art sweetener.

It is another object of the present invention to provide sweetener compounds without astringent or bitter-taste aftertaste. Also needed are sweetener compounds capable of masking the unpleasant taste (bitter/astringent/metallic) of other sweeteners. The sweetener compounds to be specified should be toxicologically safe, effective already at relatively low concentrations, well tolerated by the digestion, stable (in particular in normal food and/or pharmaceutical formulations), and easy to formulate and economical to produce.

### BRIEF DESCRIPTION OF THE INVENTION

### COMPOSITIONS

A first object of the present invention refers to a sweetener and/or sweetness enhancer composition comprising, consisting or essentially consisting of at least one of the following **Compounds 1 to 119** and preferably **Compounds 1 to 60** according to **Annex A.**

### BLENDS

In a preferred embodiment of the present invention said compositions as defined above further comprise at least one, two, three, four or more **Compounds 61 to 119** according to **Annex A.**

### METHOD (I)

Another object of the present invention refers to a method for
(i) sweetening a consumer product and/or
(ii) enhancing the sweetness of a consumer product of another sweetener, and/or
(iii) masking the unpleasant taste of another sweetener,
said sweetener preferably being a rebaudioside, and particularly being Rebaudioside D or Rebaudioside M, comprising the following steps:
(a) providing a consumer product or a sweetener different from Compounds 1 to 60 as defined in **Annex A,** and
(b) adding a composition comprising, consisting or essentially consisting of at least one, preferably two, three, four or more **Compounds 1 to 60** according to **Annex A.**

In a preferred embodiment the method makes use of a composition further comprising at least one, preferably two, three, four or more **Compounds 61 to 119** according to **Annex A.**

### METHOD (II)

Another object of the present invention refers to a method for
(i) sweetening a consumer product and/or
(ii) enhancing the sweetness of a consumer product of another sweetener, and/or
(iii) masking the unpleasant taste of another sweetener,
said sweetener preferably being a rebaudioside, and particularly being Rebaudioside D or Rebaudioside M, comprising the following steps:
(a) providing a consumer product or a sweetener different from Compounds 61 to 119 as defined in **Annex A,** and
(b) adding a composition comprising, consisting or essentially consisting of at least one, preferably two, three, four or more **Compounds 61 to 119** according to **Annex A.**

### METHOD (III)

Another object of the present invention refers to a method for
(i) sweetening a consumer product and/or
(ii) enhancing the sweetness of a consumer product of another sweetener, and/or
(iii) masking the unpleasant taste of another sweetener,
said sweetener preferably being a rebaudioside, and particularly being Rebaudioside D or Rebaudioside M, comprising the following steps:
(a) providing a consumer product or a sweetener different from Compounds 1 to 15 as defined in **Annex A,** and
(b) adding a composition comprising, consisting or essentially consisting of at least one, preferably two, three, four or more **Compounds 1 to 15** according **Annex A.**

### USE OF THE COMPOSITIONS (I)

Another object of the present invention refers to the use of at least one, preferably two, three, four or more **Compounds 1 to 60** according to **Annex A** as sweeteners and/or sweetness enhancers and/or masking agents for the unpleasant taste of another sweetener, particularly for rebaudiosides and even more preferred for Rebaudioside M. Preferred is the use of at least one, preferably two, three, four or more **Compounds 1 to 15** according to **Annex A** as sweeteners and/or sweetness enhancers and/or masking agents for the unpleasant taste of another sweetener, particularly for rebaudiosides and even more preferred for Rebaudioside M.

Said compositions may further comprise at least one, preferably two, three four or more **Compounds 61 to 119** according to **Annex A:**

### USE OF THE COMPOSITIONS (II)

Another object of the present invention refers to the use of at least one, preferably two, three, four or more **Compounds 61 to 119** according to **Annex A** as sweeteners and/or sweetness enhancers and/or masking agents for the unpleasant taste of another sweetener, particularly for rebaudiosides and even more preferred for Rebaudioside D or Rebaudioside M.

### USE OF COMPOUNDS (III)

Another object of the present invention refers to the use of at least one, preferably two, there, four or more **Compounds 61 to 69** according to **Annex A** as sweetness enhancers and/or masking agents for the unpleasant taste of another sweetener, particularly when combined with a rebaudioside, and even more particularly when combined with Rebaudioside D or Rebaudioside M.

### EXTRACTS

Another object of the present invention refers to an extract comprising one or more **Compound 1 to 119** as defined in **Annex A** obtainable by aqueous and/or alcoholic extraction, said compounds and the respected plant or microorganisms from which they are obtained from, selected from the group consisting of:

| **Compound Reference** | ***Source*** |
|---|---|
| 1 | *Momordica grosvenori* |
| 2 | *Momordica grosvenori* |
| 3 | *Momordica grosvenori* |
| 4 | *Momordica grosvenori* |
| 5 | *Momordica grosvenori* |
| 6 | *Momordica grosvenori* |
| 7 | *Momordica grosvenori* |
| 8 | *Momordica grosvenori* |
| 9 | *Momordica grosvenori* |
| 10 | *Momordica grosvenori* |
| 11 | *Momordica grosvenori* |
| 12 | *Momordica grosvenori* |
| 13 | *Momordica grosvenori* |
| 14 | *Momordica grosvenori* |
| 15 | *Momordica grosvenori* |
| 16 | *Gynostemma pentaphyllum* |
| 17 | *Momordica grosvenori* |
| 18 | *Gynostemma pentaphyllum* |
| 19 | *Momordica grosvenori* |
| 20 | *Momordica grosvenori* |
| 21 | *Momordica grosvenori* |
| 22 | *Momordica grosvenori* |
| 23 | *Momordica grosvenori* |
| 24 | *Momordica grosvenori* |
| 25 | *Momordica grosvenori* |
| 26 | *Momordica grosvenori* |
| 27 | *Momordica grosvenori* |
| 28 | *Momordica grosvenori* |
| 29 | *Momordica grosvenori* |
| 30 | *Momordica grosvenori* |
| 31 | *Momordica grosvenori* |
| 32 | *Momordica grosvenori* |
| 33 | *Momordica grosvenori* |
| 34 | *Momordica grosvenori* |
| 35 | *Momordica grosvenori* |
| 36 | *Momordica grosvenori* |
| 37 | *Momordica grosvenori* |
| 38 | *Momordica grosvenori* |
| 39 | *Momordica grosvenori* |
| 40 | *Momordica grosvenori* |
| 41 | *Hypholoma fasciculare* |
| 42 | *Gynostemma pentaphyllum* |
| 43 | *Gynostemma pentaphyllum* |
| 44 | *Gynostemma pentaphyllum* |
| 45 | *Gynostemma pentaphyllum* |
| 46 | *Gynostemma pentaphyllum* |
| 47 | *Gynostemma pentaphyllum* |
| 48 | *Gynostemma pentaphyllum* |
| 49 | *Gynostemma pentaphyllum* |
| 50 | *Gynostemma pentaphyllum* |
| 51 | *Gynostemma pentaphyllum* |
| 52 | *Gynostemma pentaphyllum* |
| 53 | *Gynostemma pentaphyllum* |
| 54 | *Gynostemma pentaphyllum* |
| 55 | *Bryonia dioica* |
| 57 | *Bryonia dioica* |
| 58 | *Pterocarya paliurus* |
| 59 | *Pterocarya paliurus* |
| 60 | *Pterocarya paliurus* |
| 61 | *Abrus precatorius* |
| 62 | *Pterocarya paliurus* |
| 63 | *Pterocarya paliurus* |
| 64 | *Pterocarya paliurus* |
| 65 | *Pterocarya paliurus* |
| 66 | *Pterocarya paliurus* |
| 67 | *Pterocarya paliurus* |
| 68 | *Pterocarya paliurus* |
| 69 | *Pterocarya paliurus* |
| 70 | *Pterocarya paliurus* |
| 71 | *Pterocarya paliurus* |
| 72 | *Pterocarya paliurus* |
| 73 | *Pterocarya paliurus* |
| 74 | *Pterocarya paliurus* |
| 75 | *Pterocarya paliurus* |
| 76 | *Myriopteron extensum* |
| 77 | *Myriopteron extensum* |
| 78 | *Myriopteron extensum* |
| 79 | *Myriopteron extensum* |
| 80 | *Myriopteron extensum* |
| 81 | *Myriopteron extensum* |
| 82 | *Myriopteron extensum* |
| 83 | *Myriopteron extensum* |
| 84 | *Myriopteron extensum* |
| 85 | *Myriopteron extensum* |
| 86 | *Myriopteron extensum* |
| 87 | *Myriopteron extensum* |
| 88 | *Myriopteron extensum* |
| 89 | *Dioscorea sp.* |
| 90 | *Abrus precatorius* |
| 91 | *Aster tataricus* |
| 92 | *Abrus precatorius* |
| 93 | *Periandra dulcis* |
| 94 | *Saponaria sp.* |
| 95 | *Dipsacus sp.* |
| 96 | *Albizia sp.* |
| 97 | *Gynostemma pentaphyllum* |
| 98 | *Gynostemma pentaphyllum* |
| 99 | *Baccharis gaudichaudiana* |
| 100 | *Baccharis gaudichaudiana* |
| 101 | *Baccharis gaudichaudiana* |
| 102 | *Phlomis betonicoides* |
| 103 | *Phlomis betonicoides* |
| 104 | *Phlomis betonicoides* |
| 105 | *Citrus aurantium* |
| 106 | *Artemisia erithmifolia* |
| 107 | *Euphorbia paralias. Rubus suavissimus* |
| 108 | *Perymenium grande* |
| 109 | *Engelhardtia crysolepis* |
| 110 | *Polypodium vulgare* |
| 111 | *Gnetum sp.* |
| 112 | *Bauhinia cardinalis* |
| 113 | *Hydrangea macrophylla* |
| 114 | *Sapindus saponaria* |
| 115 | *Fungi imperfecti* |
| 116 | *Solanum melongena* |
| 117 | *Prunus persica* |
| 118 | *Hintonia latiflora* |
| 119 | *Citrus sinensis* |

### EXTRACTION PROCESS

The extracts according to the present invention may be prepared by methods known per se, i.e. for example by aqueous, alcoholic or aqueous/alcoholic extraction of the plants or parts thereof. Suitable extraction processes are any conventional extraction processes, such as maceration, re-maceration, digestion, agitation maceration, vortex extraction, ultrasonic extraction, counter current extraction, percolation, re-percolation, evacolation (extraction under reduced pressure), diacolation and solid/liquid extraction under continuous reflux. Percolation is advantageous for industrial use.

The plant materials that are useful for obtaining the extracts may include parts of the whole plant selected from the group consisting of blossoms, fruits, buds, roots, stems, bark, seeds and/or leaves or the whole plant itself. The starting material may be mechanically size-reduced before the extraction process. Any size reduction methods known to the expert, for example freeze grinding, may be used. Preferred solvents for the extraction process are organic solvents, water (preferably hot water with a temperature above 80 °C and more particularly above 95 °C or mixtures of organic solvents and water, more particularly low molecular weight alcohols with more or less high water contents. Extraction with methanol, ethanol and water-containing mixtures thereof is particularly preferred. The extraction process is generally carried out at about 20 to about 100 °C and preferably at about 30 to about 70 °C. In one preferred embodiment, the extraction process is carried out in an inert gas atmosphere to avoid oxidation of the ingredients of the extract. This is particularly important where extraction is carried out at temperatures above 40 °C. The extraction times are selected by the expert in dependence upon the starting material, the extraction process, the extraction temperature and the ratio of solvent to raw material, etc. After the extraction process, the crude extracts obtained may optionally be subjected to other typical steps, such as for example purification, concentration and/or decoloration. If desired, the extracts thus prepared may be subjected, for example, to the selective removal of individual unwanted ingredients. The extraction process may be carried out to any degree, but is usually continued to exhaustion. Typical yields (=extract dry matter, based on the quantity of raw material used) in the extraction of the starting materials are of the order of about 5 to about 35, %, preferably about 10 to about 30 and more preferably about 15 to about 25 % b.w. - calculated on the starting materials.

Another object of the present invention refers to a process for obtaining an extract of plants listed in the tables infra rich in compounds according to formula 1 to 119 encompassing the steps of:
(a) Providing a suspension of the plant listed in the tables in water, a C₁-C₄ aliphatic alcohol or their mixtures
(b) Subjecting said suspension to extraction using water, a C₁-C₄ aliphatic alcohol or their mixtures at temperatures of about 30 to about 70 °C, and optionally
(c) Separating off the solvent.

It should be noted that the content in the extract of each of the compounds **1** to **119** differs from batch to batch depending on the used raw material.

### CONSUMER PRODUCTS

The preparation of the present invention comprising at least one sweetener **Compound of formula 1 to 119** and can be used to impart a desirable sweetness and/ or flavor to a variety of oral and food compositions and pharmaceutical compositions, such as beverages, edible foodstuff, dentifrices, lipsticks and the like, which may or may not be ingestible, with or without the use of other flavorants and sweeteners. The present invention also relates to a variety of oral and food products and the like embodying at least one compound of formula **1** to **119** as sweetener and/ or flavoring agent.

The sweeteners or sweetness enhancers of this invention find application in the wide range of edible substances generally, primarily in food compositions such as candies, confections and processed foods, and beverages such as beer and soft drinks. It is also well suited for imparting a sweet flavor to other edible substances such as medicines, toothpaste, adhesives for stamps and envelopes, animal feeds and baits and the like. These examples are given solely for illustration and it is not wished to limit the scope of this invention to sweetening any particular type or types of edible materials. As a general rule, the present sweetener may be used in any application where a sweet taste is desired. The present sweetener may be used alone or in combination with other sweeteners, nutritive or nonnutritive. Also, if desired, binders or diluents may be added to the sweetener. This is not usually necessary, however, as the sweetener is a solid having excellent handling properties. This makes mixing the sweetener with an edible substance a simple conventional operation. The sweetener may be mixed with the edible substance as a solid or as a solution, if desired.

As indicated infra the inventions also refers to the use of a sweetener compound of formula **1** to **119** to sweet or enhance the sweeting effect in compositions or preparations which are administered to an individual in an effective amount sufficient to produce the desired degree of sweetness.

Thus the invention further relates to a method for providing a sweetening effect and/or an enhanced sweetening effect in compositions, comprising administering to an individual a sweetener compound of formula (I) in an effective amount sufficient to produce the desired degree of sweetness.

The effective amount is preferably from 1 ppm to 2000 ppm, preferably about 5 to about 1.000 ppm, more preferably about 10 to about 500 ppm and most preferably about 50 to about 100 ppm based on the total weight of the composition and the total sum of all compounds 1 to 122.

The food, oral and pharmaceutical compositions will be further described in detail.

### FOOD COMPOSITIONS

Another object of the present invention refers to a food composition, comprising the sweetener composition or the extract as disclosed above.

Food compositions according to the invention are any preparations or compositions which are suitable for consumption and are used for nutrition or enjoyment purposes, and are generally products which are intended to be introduced into the human or animal oral cavity, to remain there for a certain time and then either be eaten (e.g. ready-to-eat foodstuffs or feeds, see also herein below) or removed from the oral cavity again (e.g. chewing gums). Such products include any substances or products which in the processed, partially processed or unprocessed state are to be ingested by humans or animals. They also include substances which are added to orally consumable products during their manufacture, preparation or treatment and which are intended to be introduced into the human or animal oral cavity.

The food compositions according to the invention also include substances which in the unchanged, treated or prepared state are to be swallowed by a human or animal and then digested; in this respect, the orally consumable products according to the invention also include casings, coatings or other encapsulations which are to be swallowed at the same time or which may be expected to be swallowed. The expression "orally consumable product" covers ready-to-eat foodstuffs and feeds, that is to say foodstuffs or feeds that are already complete in terms of the substances that are important for the taste. The expressions "ready-to-eat foodstuff" and "ready-to-eat feed" also include drinks as well as solid or semi-solid ready-to-eat foodstuffs or feeds. Examples which may be mentioned are frozen products, which must be thawed and heated to eating temperature before they are eaten. Products such as yoghurt or ice-cream as well as chewing gums or hard caramels are also included among the ready-to-eat foodstuffs or feeds.

Preferred food compositions according to the invention also include "semi-finished products". Within the context of the present text, a semi-finished product is to be understood as being an orally consumable product which, because of a very high content of flavorings and taste-imparting substances, is unsuitable for use as a ready-to-eat orally consumable product (in particular foodstuff or feed). Only by mixing with at least one further constituent (e.g. by reducing the concentration of the flavorings and taste-imparting substances in question) and optionally further process steps (e.g. heating, freezing) is the semi-finished product converted into a ready-to-eat orally consumable product (in particular foodstuff or feed). Examples of semi-finished products which may be mentioned here are

Food composition according to the invention preferably comprises one or more preparations for nutrition or enjoyment purposes. These include in particular (reduced-calorie) baked goods (e.g. bread, dry biscuits, cakes, other baked articles), confectionery (e.g. chocolates, chocolate bars, other products in bar form, fruit gums, dragées, hard and soft caramels, chewing gum), non-alcoholic drinks (e.g. cocoa, coffee, green tea, black tea, (green, black) tea drinks enriched with (green, black) tea extracts, rooibos tea, other herbal teas, fruit-containing soft drinks, isotonic drinks, refreshing drinks, nectars, fruit and vegetable juices, fruit or vegetable juice preparations), instant drinks (e.g. instant cocoa drinks, instant tea drinks, instant coffee drinks), meat products (e.g. ham, fresh sausage or raw sausage preparations, spiced or marinated fresh or salt meat products), eggs or egg products (dried egg, egg white, egg yolk), cereal products (e.g. breakfast cereals, muesli bars, precooked ready-to-eat rice products), dairy products (e.g. full-fat or reduced-fat or fat-free milk drinks, rice pudding, yoghurt, kefir, cream cheese, soft cheese, hard cheese, dried milk powder, whey, butter, buttermilk, partially or completely hydrolysed milk-protein-containing products), products made from plant protein such as for example peas or oat or other plant protein fractions (e.g. soy milk and products produced therefrom, drinks containing isolated or enzymatically treated plant protein, drinks containing plant flour, preparations containing plant lecithin, fermented products such as tofu or tempeh or products produced therefrom and mixtures with fruit preparations and optionally flavors), fruit preparations (e.g. jams, sorbets, fruit sauces, fruit fillings), vegetable preparations (e.g. ketchup, sauces, dried vegetables, frozen vegetables, precooked vegetables, boiled-down vegetables), snacks (e.g. baked or fried potato crisps or potato dough products, maize- or groundnut-based extrudates), fat- and oil-based products or emulsions thereof (e.g. mayonnaise, remoulade, dressings, in each case full-fat or reduced-fat), other ready-made dishes and soups (e.g. dried soups, instant soups, precooked soups), spices, spice mixtures and in particular seasonings which are used, for example, in the snacks field, sweetener preparations, tablets or sachets, other preparations for sweetening or whitening drinks or other foods. The preparations within the scope of the invention can also be used in the form of semi-finished products for the production of further preparations for nutrition or enjoyment purposes. The preparations within the scope of the invention can also be in the form of capsules, tablets (uncoated and coated tablets, e.g. enteric coatings), dragées, granules, pellets, solids mixtures, dispersions in liquid phases, in the form of emulsions, in the form of powders, in the form of solutions, in the form of pastes, or in the form of other preparations which can be swallowed or chewed, and in the form of food supplements or meat analogues.

The preparations can also be in the form of capsules, tablets (uncoated and coated tablets, e.g. enteric coatings), dragées, granules, pellets, solids mixtures, dispersions in liquid phases, in the form of emulsions, in the form of powders, in the form of solutions, in the form of pastes, or in the form of other preparations which can be swallowed or chewed, for example in the form of food supplements.

The semi-finished products are generally used for the production of ready-to-use or ready-to-eat preparations for nutrition or enjoyment purposes.

Further constituents of a ready-to-eat preparation or semi-finished product for nutrition or enjoyment purposes can be conventional base substances, auxiliary substances and additives for foods or enjoyment foods, for example water, mixtures of fresh or processed, vegetable or animal base or raw substances (e.g. raw, roast, dried, fermented, smoked and/or boiled meat, bone, cartilage, fish, vegetables, herbs, nuts, vegetable juices, vegetable pastes or mixtures thereof), digestible or non-digestible carbohydrates (e.g. sucrose, maltose, fructose, glucose, dextrins, amylose, amylopectin, inulin, xylans, cellulose, tagatose), sugar alcohols (e.g. sorbitol, erythritol), natural or hardened fats (e.g. tallow, lard, palm fat, cocoa fat, hardened vegetable fat), oils (e.g. sunflower oil, groundnut oil, maize germ oil, olive oil, fish oil, soya oil, sesame oil), fatty acids or their salts (e.g. potassium stearate), proteinogenic or non-proteinogenic amino acids and related compounds (e.g. γ-aminobutyric acid, taurine), peptides (e.g. glutathione), natural or processed proteins (e.g. gelatin), enzymes (e.g. peptidases), nucleic acids, nucleotides, taste correctors for unpleasant taste impressions, further taste modulators for further, generally not unpleasant taste impressions, other taste-modulating substances (e.g. inositol phosphate, nucleotides such as guanosine monophosphate, adenosine monophosphate or other substances such as sodium glutamate or 2-phenoxypropionic acid), emulsifiers (e.g. lecithins, diacylglycerols, gum arabic), stabilisers (e.g. carrageenan, alginate), preservatives (e.g. benzoic acid and its salts, sorbic acid and its salts), antioxidants (e.g. tocopherol, ascorbic acid), chelators (e.g. citric acid), organic or inorganic acidifying agents (e.g. acetic acid, phosphoric acid), additional bitter substances (e.g. quinine, caffeine, limonene, amarogentine, humulone, lupulone, catechols, tannins), substances that prevent enzymatic browning (e.g. sulfite, ascorbic acid), ethereal oils, plant extracts, natural or synthetic colourings or colouring pigments (e.g. carotinoids, flavonoids, anthocyans, chlorophyll and derivatives thereof), spices, trigeminally active substances or plant extracts containing such trigeminally active substances, synthetic, natural or nature-identical flavorings or odorants as well as odour correctors.

Food compositions according to the invention, for example those in the form of preparations or semi-finished products, preferably comprise a flavor composition in order to complete and refine the taste and/or odour. A preparation can comprise as constituents a solid carrier and a flavor composition. Suitable flavor compositions comprise, for example, synthetic, natural or nature-identical flavorings, odorants and taste-imparting substances, reaction flavorings, smoke flavorings or other flavor-giving preparations (e.g. protein (partial) hydrolysates, preferably protein (partial) hydrolysates having a high arginine content, barbecue flavorings, plant extracts, spices, spice preparations, vegetables and/or vegetable preparations) as well as suitable auxiliary substances and carriers. Particularly suitable here are the flavor compositions or constituents thereof which produce a roasted, meaty (in particular chicken, fish, seafood, beef, pork, lamb, mutton, goat), vegetable-like (in particular tomato, onion, garlic, celery, leek, mushroom, aubergine, seaweed), spicy (in particular black and white pepper, cardamom, nutmeg, pimento, mustard and mustard products), fried, yeast-like, boiled, fatty, salty and/or pungent flavor impression and accordingly can enhance the spicy impression. The flavor compositions generally comprise more than one of the mentioned ingredients.

The food compositions of the present invention are preferably selected from the group comprising
- confectionery, preferably reduced-calorie or calorie-free confectionery, preferably selected from the group comprising muesli bar products, fruit gums, dragées, hard caramels and chewing gum,
- non-alcoholic drinks, preferably selected from the group comprising green tea, black tea, (green, black) tea drinks enriched with (green, black) tea extracts, rooibos tea, other herbal teas, fruit-containing low-sugar or sugar-free soft drinks, isotonic drinks, nectars, fruit and vegetable juices, fruit and vegetable juice preparations,
- alcoholic drinks, preferably selected from the group comprising alcoholic beverages to which herbal extracts or fruit or vegetable juices or flavorings have been added (alcopops)
- instant drinks, preferably selected from the group comprising instant (green, black, rooibos, herbal) tea drinks,
- cereal products, preferably selected from the group comprising low-sugar and sugar-free breakfast cereals and muesli bars,
- dairy products, preferably selected from the group comprising reduced-fat and fat-free milk drinks, yoghurt, kefir, whey, buttermilk and ice-cream,
- products made from soy protein or other soybean fractions, preferably selected from the group comprising soy milk, products produced from soy milk, drinks containing isolated or enzymatically treated soy protein, drinks containing soy flour, preparations containing soy lecithin, products produced from preparations containing soy lecithin and mixtures with fruit preparations and optionally flavors,
- sweetener preparations, tablets and sachets,
- sugar-free dragées,
- ice-cream, with or without milk-based constituents, preferably sugar-free.

### FOOD ADDITIVES

The food compositions according to the present invention may further comprise components selected from the group consisting of additional sweeteners or sweet-tasting compounds, aroma compounds, flavoring compounds and their mixtures.

### Aroma or flavoring compounds

Aroma compounds and flavoring agents are well known in the art can be added to the flavor compositions of the invention. These flavoring agents can be chosen from synthetic flavoring liquid and/or oils derived from plants leaves, flowers, fruits and so forth, and combinations thereof. Representative flavoring liquids include: artificial, natural or synthetic fruit flavors such as eucalyptus, lemon, orange, banana, grape, lime, apricot and grapefruit oils and fruit essences including apple, strawberry, cherry, orange, pineapple and so forth; bean and nut derived flavors such as coffee, cocoa, cola, peanut, almond and so forth; and root derived flavors such as licorice or ginger.

The flavoring agent is preferably selected from the group consisting of essential oils and extracts, tinctures and balsams, such as, for example, anisole, basil oil, bergamot oil, bitter almond oil, camphor oil, citronella oil, lemon oil; Eucalyptus citriodora oil, eucalyptus oil, fennel oil, grapefruit oil, camomile oil, spearmint oil, caraway oil, lime oil, mandarin oil, nutmeg oil (in particular nutmeg blossom oil = maces oil, mace oil), myrrh oil, clove oil, clove blossom oil, orange oil, oregano oil, parsley (seed) oil, peppermint oil, rosemary oil, sage oil (clary sage, Dalmatian or Spanish sage oil), star aniseed oil, thyme oil, vanilla extract, juniper oil (in particular juniper berry oil), wintergreen oil, cinnamon leaf oil; cinnamon bark oil, and fractions thereof, or constituents isolated therefrom.

It is of particular advantage if the flavored composition according to the invention comprises at least one flavoring agent, preferably two, three, four, five, six, seven, eight or more flavoring agents chosen from the following group: menthol (preferably I-menthol and/or racemic menthol), anethole, anisole, anisaldehyde, anisyl alcohol, (racemic) neomenthol, eucalyptol (1,8-cineol), menthone (preferably L-menthone), isomenthone (preferably D-isomenthone), isopulegol, menthyl acetate (preferably L-menthyl acetate), menthyl propionate, carvone (preferably (-)-carvone, optionally as a constituent of a spearmint oil), methyl salicylate (optionally as a constituent of a wintergreen oil), eugenol acetate, isoeugenol methyl ether, beta-homocyclocitral, eugenol, isobutyraldehyde, 3-octanol, dimethyl sulfide, hexanol, hexanal, trans-2-hexenal, cis-3-hexenol, 4-terpineol, piperitone, linalool, 8-ocimenyl acetate, isoamyl alcohol, isovaleraldehyde, alpha-pinene, beta-pinene, limonene (preferably D-limonene, optionally as a constituent of an essential oil), piperitone, trans-sabinene hydrate, menthofuran, caryophyllene, germacrene D, cinnamaldehyde, mint lactone, thymol, gamma-octalactone, gamma-nonalactone, gamma-decalactone, (1,3E,5Z)-undecatriene, 2-butanone, ethyl formate, 3-octyl acetate, isoamyl isovalerate, cis- and trans-carvyl acetate, p-cymol, damascenone, damascone, cis-rose oxide, trans-rose oxide, fenchol, acetaldehyde diethyl acetal, 1-ethoxyethyl acetate, cis-4-heptenal, cis-jasmone, methyl dihydrojasmonate, 2'-hydroxypropiophenone, menthyl methyl ether, myrtenyl acetate, 2-phenylethyl alcohol, 2-phenylethyl isobutyrate, 2-phenylethyl isovalerate, geraniol, nerol and viridiflorol.

In particular preferred aroma or flavoring compounds encompass menthol, cineol, eugenol, thymol, cinnamic aldehyde, peppermint oil, spearmint oil, eucalyptus oil, thyme oil, cinnamon oil, clove oil, spruce needle oil, fennel oil, sage oil, aniseed oil, star anise oil, chamomile oil, and caraway oil, and their mixtures.

### SWEETENERS AND SWEET-TASTING SUBSTANCES

The term "sweeteners" here denotes substances having a relative sweetening power of at least 25, based on the sweetening power of sucrose (which accordingly has a sweetening power of 1). Sweeteners to be used in an orally consumable product (in particular foodstuff, feed or medicament) according to the invention (a) are preferably non-cariogenic and/or have an energy content of not more than 5 kcal per gram of the orally consumable product.

It should be noted that the Compounds 1 to 119 are capable of enhancing the sweetness of said sweeteners and sweet-tasting substances significantly. Preferably, one or more of the compound of formula 1 to 122 is used in an amount from 1 ppm to 2000 ppm by weight, based on the total weight of the composition and base on the total sum of all compounds of formula1 to 119, if more than one compound of formula 1 to 119 is used. More preferably, the sweetener compounds of formula 1 to 119 are used in an amount from 10 ppm to 1000 ppm by weight, most preferably in an amount of 20 ppm to 500 ppm by weight, based on the total weight of the composition and base on the total sum of all compounds of formula (I), if more than one compound of formula 1 to 119 is used.

Advantageous sweeteners in a preferred orally consumable product (in particular foodstuff, feed or medicament) according to the invention are selected from the following groups :

**Naturally occurring sweeteners,** preferably selected from the group comprising
- miraculin, monellin, mabinlin, thaumatin, curculin, brazzein, pentaidin, D-phenylalanine, D-tryptophan, and extracts or fractions obtained from natural sources, comprising those amino acids and/or proteins, and the physiologically acceptable salts of those amino acids and/or proteins, in particular the sodium, potassium, calcium or ammonium salts;
- neohesperidin dihydrochalcone, naringin dihydrochalcone, stevioside, steviolbioside, rebaudiosides, in particular rebaudioside A, rebaudioside B, rebaudioside C, rebaudioside D, rebaudioside E, rebaudioside F, rebaudioside G, rebaudioside H, dulcosides and rubusoside, suavioside A, suavioside B, suavioside G, suavioside H, suavioside I, suavioside J, baiyunoside 1, baiyunoside 2, phlomisoside 1, phlomisoside 2, phlomisoside 3 and phlomisoside 4, abrusoside A, abrusoside B, abrusoside C, abrusoside D, osladin, polypodoside A, strogin 1, strogin 2, strogin 4, selligueain A, dihydroquercetin 3-acetate, perillartin, telosmoside A₁₅, periandrin I-V, pterocaryosides, cyclocaryosides, mukuroziocides, trans-anethole, trans-cinnamaldehyde, bryosides, bryonosides, bryonodulcosides, carnosiflosides, scandenosides, trilobatin, phloridzin, dihydroflavanols, hematoxylin, cyanin, chlorogenic acid, albiziasaponin, telosmosides, gaudichaudioside, mogrosides, mogroside V, hernandulcins, monatin, phyllodulcin, glycyrrhetinic acid and derivatives thereof, in particular glycosides thereof such as glycyrrhizine, and the physiologically acceptable salts of those compounds, in particular the sodium, potassium, calcium or ammonium salts;
- extracts or concentrated fractions of the extracts, selected from the group comprising Thaumatococcus extracts (katamfe plant), extracts from *Stevia rebaudiana,* extracts from *Momordica* or *Siratia grosvenorii* (Luo-Han-Guo), extracts from *Glycyrrhiza* ssp. (in particular *Glycyrrhiza glabra*), extracts from *Rubus* ssp. (in particular *Rubus suavissimus*), and extracts from *Lippia dulcis;*

**Synthetic sweet-tasting substances,** preferably selected from the group comprising magap, sodium cyclamate or other physiologically acceptable salts of cyclamic acid, acesulfame K or other physiologically acceptable salts of acesulfame, neohesperidin dihydrochalcone, naringin dihydrochalcone, saccharin, saccharin sodium salt, aspartame, superaspartame, neotame, alitame, advantame, perillartin, sucralose, lugduname, carrelame, sucrononate and sucrooctate.

Suitable sweet-tasting substances, including natural sources of these substances such as for example
- sweet-tasting carbohydrates or sugars (e.g. sucrose (synonymous with saccharose), trehalose, lactose, maltose, melizitose, raffinose, palatinose, lactulose, D-fructose, D-glucose, D-galactose, L-rhamnose, D-sorbose, D-mannose, D-tagatose, D-arabinose, L-arabinose, D-ribose, D-glyceraldehyde, maltodextrin) or
- vegetable preparations containing predominantly these carbohydrates (e.g. from sugar beet (Beta vulgaris ssp., sugar fractions, sugar syrup, molasses), from sugar cane (Saccharum officinarum ssp., e.g. molasses, sugar syrups), from sugar maple (Acer ssp.), from agave (agave thick juice),
- synthetic/enzymatic hydrolysates of starch or sucrose (e.g. invert sugar syrup, highly enriched fructose syrups made from corn starch),
- fruit concentrates (e.g. from apples or pears, apple syrup, pear syrup),
- sugar alcohols (e.g. erythritol, threitol, arabitol, ribitol, xylitol, Sorbitol, mannitolol, dulcitol, lactitol),
- proteins (e.g. miraculin, monellin, thaumatin, curculin, brazzein),
- artificial sweeteners (magap, sodiumcyclamate, acesulfame K, neohesperidin dihydrochalcone, saccharin sodium salt, aspartame®, superaspartame, neotame, alitame, sucralose, lugduname, carrelame, sucrononate, sucrooctate, monatin),
- certain sweet-tasting amino acids (glycine, D-leucine, D-threonine, D-asparagine, D-phenylalanine, D-tryptophan, L-proline),
- other sweet-tasting low-molecular substances, e.g. rebaudioside, stevioside, mogrosides, hernandulcin, phyllodulcin, dihydrochalcone glycosides, glycyrrhizin, glycyrrhetinic acid ammonium salt or other glycyrrhetinic acid derivatives,
- extracts from sweet tasting plants, in particular Momordica grosvenori [Luo Han Guo] Hydrangea macrophylla, Stevia rebaudiana, Rubus suavissimus, Polypodium vulgare, Abrus precatorius, Pterocarya paliurus, Baccharis gaudichaudiana, Albizia myriophylla, Bryonia dioica, Phlomis betonicoides, Hemsleya carnosiflora, Lippia dulcis, Glycyrrhiza sp. (liquorice), Abrus sp., Myriopteron extensum, Gynostemma pentaphyllum, Periandra dulcis or individual sweet tasting substances isolated from those plants.

### THICKENERS

Advantageous thickeners in a preferred orally consumable product (in particular foodstuff, feed or medicament) according to the invention are selected from the group comprising: crosslinked polyacrylic acids and derivatives thereof, polysaccharides and derivatives thereof, such as xanthan gum, agar-agar, alginates or tyloses, cellulose derivatives, for example carboxymethylcellulose or hydroxycarboxymethylcellulose, fatty alcohols, monoglycerides and fatty acids, polyvinyl alcohol and polyvinylpyrrolidone.

Preference is given according to the invention to an orally consumable product (in particular foodstuff or feed) which comprises milk thickened with lactic acid bacteria and/or cream thickened with lactic acid bacteria and which preferably is selected from the group comprising yoghurt, kefir and quark.

A food composition according to the invention comprising milk thickened with lactic acid bacteria and/or cream thickened with lactic acid bacteria is advantageously an orally consumable product which comprises a probiotic, wherein the probiotic is preferably selected from the group comprising Bifidobacterium animalis subsp. lactis BB-12, Bifidobacterium animalis subsp. lactis DN-173 010, Bifidobacterium animalis subsp. lactis HN019, Lactobacillus acidophilus LA5, Lactobacillus acidophilus NCFM, Lactobacillus johnsonii La1, Lactobacillus casei immunitass/defensis, Lactobacillus casei Shirota (DSM 20312), Lactobacillus casei CRL431, Lactobacillus reuteri (ATCC 55730) and Lactobacillus rhamnosus (ATCC 53013).

### ADDITIVES FOR CHEWING GUMS

Particular preference is given to an orally consumable product (in particular foodstuff, feed or medicament) according to the invention that is a chewing gum and comprises a chewing-gum base. The chewing-gum base is preferably selected from the group comprising chewing-gum or bubble-gum bases. The latter are softer, so that gum bubbles can also be formed therewith. Preferred chewing-gum bases according to the invention include, in addition to the natural resins or the natural latex chicle that are traditionally used, elastomers such as polyvinyl acetate (PVA), polyethylene, (low or medium molecular weight) polyisobutene (PIB), polybutadiene, isobutene-isoprene copolymers (butyl rubber), polyvinyethyl ether (PVE), polyvinylbutyl ether, copolymers of vinyl esters and vinyl ethers, styrene-butadiene copolymers (styrene-butadiene rubber, SBR) or vinyl elastomers, for example based on vinyl acetate/vinyl laurate, vinyl acetate/vinyl stearate or ethylene/vinyl acetate, as well as mixtures of the mentioned elastomers, as described, for example, in EP 0 242 325, US 4,518,615, US 5,093,136, US 5,266,336, US 5,601,858 or US 6,986,709. In addition, chewing-gum bases that are preferably to be used according to the invention preferably comprise further constituents such as, for example, (mineral) fillers, plasticisers, emulsifiers, antioxidants, waxes, fats or fatty oils, such as, for example, hardened (hydrogenated) vegetable or animal fats, mono-, di- or tri-glycerides. Suitable (mineral) fillers are, for example, calcium carbonate, titanium dioxide, silicon dioxide, talcum, aluminium oxide, dicalcium phosphate, tricalcium phosphate, magnesium hydroxide and mixtures thereof. Suitable plasticisers, or agents for preventing adhesion (detackifiers), are, for example, lanolin, stearic acid, sodium stearate, ethyl acetate, diacetin (glycerol diacetate), triacetin (glycerol triacetate), triethyl citrate. Suitable waxes are, for example, paraffin waxes, candelilla wax, carnauba wax, microcrystalline waxes and polyethylene waxes. Suitable emulsifiers are, for example, phosphatides such as lecithin, mono- and diglycerides of fatty acids, for example glycerol monostearate.

Chewing gums according to the invention (in particular as disclosed above) preferably comprise constituents such as sugars of different types, sugar substitutes, other sweet-tasting substances, sugar alcohols (in particular Sorbitol, xylitol, mannitol), ingredients having a cooling effect, taste correctors for unpleasant taste impressions, further taste-modulating substances (e.g. inositol phosphate, nucleotides such as guanosine monophosphate, adenosine monophosphate or other substances such as sodium glutamate or 2-phenoxypropionic acid), humectants, thickeners, emulsifiers, stabilisers, odor correctors and flavors (e.g. eucalyptus-menthol, cherry, strawberry, grapefruit, vanilla, banana, citrus, peach, blackcurrant, tropical fruits, ginger, coffee, cinnamon, combinations (of the mentioned flavors) with mint flavors as well as spearmint and peppermint on their own). The combination *inter alia* of the flavors with further substances that have cooling, warming and/or mouth-watering properties is of particular interest.

### VITAMINS

In another embodiment of the present invention the compositions may include vitamins (component e1). Vitamins have diverse biochemical functions. Some have hormone-like functions as regulators of mineral metabolism (e.g., vitamin D), or regulators of cell and tissue growth and differentiation (e.g., some forms of vitamin A). Others function as antioxidants (e.g., vitamin E and sometimes vitamin C). The largest numberof vitamins (e.g. B complex vitamins) act as precursors for enzyme cofactors, that support the catalytic function of enzymes in metabolism. In this role, vitamins may be tightly bound to enzymes as part of prosthetic groups: For example, biotin is part of enzymes involved in making fatty acids. Vitamins may also be less tightly bound to enzyme catalysts as coenzymes, detachable molecules that function to carry chemical groups or electrons between molecules. For example, folic acid carries various forms of carbon group-methyl, formyl, and methylene - in the cell. Although these roles in assisting enzyme-substrate reactions are vitamins' best-known function, the other vitamin functions are equally important. In the course of the present invention suitable vitamins are selected from the group consisting of
- Vitamin A (retinol, retinal, beta carotene),
- Vitamin B₁ (thiamine),
- Vitamin B₂ (riboflavin),
- Vitamin B₃ (niacin, niacinamide),
- Vitamin B₅ (panthothenic acid),
- Vitamin B₆ (pyridoxine, pyridoxamine, paridoxal),
- Vitamin B₇ (biotin),
- Vitamin B₉ (folic acid, folinic acid),
- Vitamin B₁₂ (cyanobalamin, hydoxycobalmin, methylcobalmin),
- Vitamin C (ascorbic acid),
- Vitamin D (cholecalciferol),
- Vitamin E (tocopherols, tocotrienols), and
- Vitamin K (phyolloquinone, menaquinone).

The preferred vitamins are ascorbic acid and tocopherols. Said vitamins may be present in the food composition in amounts of about 0.1 to about 5 % b.w., and preferably about 0.5 to about 1 % b.w.

### ORAL COMPOSITIONS

Another object of the present invention refers to an oral composition, comprising the sweetener composition or the extract as disclosed above. These compositions may further contain additional sweeteners or sweet-tasting compounds, aroma compounds, flavoring compounds and their mixtures as already described above.

Typical examples for non-food oral compositions encompass products for cleaning and protecting teeth and refreshing the oral cavity.

The oral compositions of the present invention typically comprise an abrasive system (abrasive or polishing agent), such as e.g. silicas, calcium carbonates, calcium phosphates, aluminium oxides and/or hydroxyapatites, surface-active substances, such as e.g. sodium lauryl sulfate, sodium lauryl sarcosinate and/or cocamidopropyl betaine, moisture-retaining agents, such as e.g. glycerol and/or Sorbitol, thickening agents, such as e.g. carboxymethylcellulose, polyethylene glycols, carrageenan and/or Laponite®, sweeteners, such as e.g. saccharin, flavor correctants for unpleasant taste impressions, flavor correctants for further, as a rule not unpleasant taste impressions, flavor-modulating substances (e.g. inositol phosphate, nucleotides, such as guanosine monophosphate, adenosine monophosphate or other substances, such as sodium glutamate or 2-phenoxypropionic acid), cooling active compounds, such as e.g. menthol derivatives (e.g. L-menthyl lactate, L-menthyl alkyl carbonates, menthone ketals, menthanecarboxylic acid amides), 2,2,2-trialkylacetic acid amides (e.g. 2,2-diisopropylpropionic acid methylamide), icilin and icilin derivatives, stabilizers and active compounds, such as e.g. sodium fluoride, sodium monofluorophosphate, tin difluoride, quaternary ammonium fluorides, zinc citrate, zinc sulfate, tin pyrophosphate, tin dichloride, mixtures of various pyrophosphates, triclosan, chlorhexidine, cetylpyridinium chloride, aluminium lactate, potassium citrate, potassium nitrate, potassium chloride, strontium chloride, hydrogen peroxide, aromas, sodium bicarbonate and/or odour correctants.

Formulations or products according to the invention in the form of chewing gums or, in particular, dental care chewing gums comprise chewing gum bases which comprise elastomers, such as, for example, polyvinyl acetates (PVA), polyethylenes, (low or medium molecular weight) polyisobutenes (PIB), polybutadienes, isobutene-isoprene copolymers (butyl rubber), polyvinyl ethyl ethers (PVE), polyvinyl butyl ethers, copolymers of vinyl esters and vinyl ethers, styrene/butadiene copolymers (styrene/butadiene rubber, SBR) or vinyl elastomers, e.g. based on vinyl acetate/ vinyl laurate, vinyl acetate/vinyl stearate or ethylene/vinyl acetate, and mixtures of the elastomers mentioned, as described, for example, in EP 0 242 325, US 4,518,615, US 5,093,136, US 5,266,336 US 5,601,858 or US 6,986,709. In addition, chewing gum bases comprise further constituents, such as, for example, sugars, sugar substitutes or sweet-tasting substances in particular those described in WO 2009/21558, (mineral) fillers, plasticizers, emulsifiers, antioxidants, waxes, fats or fatty oils, such as, for example, hardened (hydrogenated) plant or animal fats, and mono-, di- or triglycerides. Suitable (mineral) fillers are, for example, calcium carbonate, titanium dioxide, silicon dioxide, talc, aluminium oxide, dicalcium phosphate, tricalcium phosphate, magnesium hydroxide and mixtures thereof. Suitable plasticizers or agents for preventing sticking (detackifiers) are, for example, lanolin, stearic acid, sodium stearate, ethyl acetate, diacetin (glycerol diacetate), triacetin (glycerol triacetate) and triethyl citrate. Suitable waxes are, for example, paraffin waxes, candelilla wax, carnauba wax, microcrystalline waxes and polyethylene waxes. Suitable emulsifiers are, for example, phosphatides, such as lecithin, and mono- and diglycerides of fatty acids, e.g. glycerol monostearate.

Formulations or products according to the invention (in particular those which are in the form of an oral care formulation or product or in the form of a formulation) preferably additionally comprise one or more aroma and/or flavoring substances, such as essential oils and extracts, tinctures and balsams, such as, for example, anisole, basil oil, bergamot oil, bitter almond oil, camphor oil, citronella oil, lemon oil; Eucalyptus citriodora oil, eucalyptus oil, fennel oil, grapefruit oil, camomile oil, spearmint oil, caraway oil, lime oil, mandarin oil, nutmeg oil (in particular nutmeg blossom oil = maces oil, mace oil), myrrh oil, clove oil, clove blossom oil, orange oil, oregano oil, parsley (seed) oil, peppermint oil, rosemary oil, sage oil (clary sage, Dalmatian or Spanish sage oil), star aniseed oil, thyme oil, vanilla extract, juniper oil (in particular juniper berry oil), wintergreen oil, cinnamon leaf oil; cinnamon bark oil, and fractions thereof, or constituents isolated therefrom.

It is of particular advantage if said formulations or products comprise at least one or more aroma substances, chosen from the following group: menthol (preferably I-menthol and/or racemic menthol), anethole, anisole, anisaldehyde, anisyl alcohol, (racemic) neomenthol, eucalyptol (1,8-cineol), menthone (preferably L-menthone), isomenthone (preferably D-isomenthone), isopulegol, menthyl acetate (preferably L-menthyl acetate), menthyl propionate, carvone (preferably (-)-carvone, optionally as a constituent of a spearmint oil), methyl salicylate (optionally as a constituent of a wintergreen oil), eugenol acetate, isoeugenol methyl ether, beta-homocyclocitral, eugenol, isobutyraldehyde, 3-octanol, dimethyl sulfide, hexanol, hexanal, trans-2-hexenal, cis-3-hexenol, 4-terpineol, piperitone, linalool, 8-ocimenyl acetate, isoamyl alcohol, isovaleraldehyde, alpha-pinene, beta-pinene, limonene (preferably D-limonene, optionally as a constituent of an essential oil), piperitone, trans-sabinene hydrate, menthofuran, caryophyllene, germacrene D, cinnamaldehyde, mint lactone, thymol, gamma-octalactone, gamma-nonalactone, gamma-decalactone, (1,3E,5Z)-undecatriene, 2-butanone, ethyl formate, 3-octyl acetate, isoamyl isovalerate, cis- and trans-carvyl acetate, p-cymol, damascenone, damascone, cis-rose oxide, trans-rose oxide, fenchol, acetaldehyde diethyl acetal, 1-ethoxyethyl acetate, cis-4-heptenal, cis-jasmone, methyl dihydrojasmonate, 2'-hydroxypropiophenone, menthyl methyl ether, myrtenyl acetate, 2-phenylethyl alcohol, 2-phenylethyl isobutyrate, 2-phenylethyl isovalerate, geraniol, nerol and viridiflorol.

### PHARMACEUTICAL COMPOSITIONS

Another object of the present invention refers to a pharmaceutical composition, comprising the sweetener composition or the extract as disclosed above. These compositions may further contain additional sweeteners or sweet-tasting compounds, aroma compounds, flavoring compounds and their mixtures as already described above.

Pharmaceutical compositions according to the present invention may include similar additives as already explained for the food and oral compositions, such as aroma and flavors. Pharmaceutical compositions may further include, oil bodies or emulsifiers and in particular co-actives supporting the beneficial properties of the pharmaceutical active agent. Therefore, the border between food compositions and pharmaceutical compositions is in flow and it should be understood that components cited for one application are recommended for the other *mutatis-mutandis* without literal repetition.

### ANNEX A

### INVENTIVE ACTIVES 1 TO 119

| **C** | **Formula** | **Name** | **Structure** | **Biomaterial Source** |
|---|---|---|---|---|
| | **MW** | | | |
| | **Cas-No.** | | | |
| **1** | C48H82O19 | Mogroside III E | 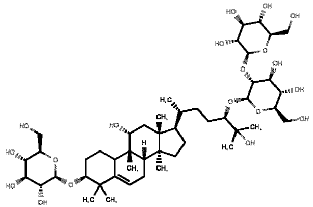 | *Momordica grosvenori* |
| | 963,165 | | | |
| | 88901-37-5 | | | |
| **2** | C48H82O19 | Mogroside III A1 | 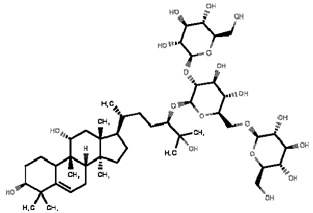 | *Momordica grosvenori* |
| | 963,165 | | | |
| | 88901-42-2 | | | |
| **3** | C42H72O14 | Mogroside II A | 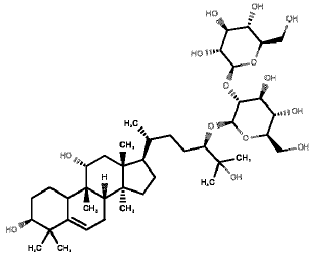 | *Momordica grosvenori* |
| | 801,024 | | | |
| | 1613527-65-3 | | | |
| **4** | C60H102O28 | no common name | 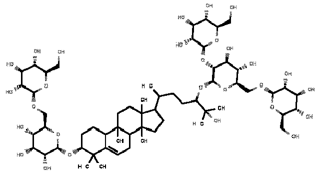 | *Momordica grosvenori* |
| | 1271,448 | | | |
| | 1707161-17-8 | | | |
| **5** | C60H102O29 | Isomogroside V | 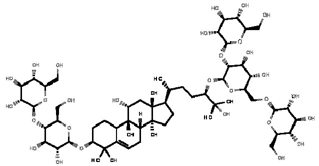 | *Momordica grosvenori* |
| | 1287,447 | | | |
| | 1126032-65-2 | | | |
| **6** | C66H112O34 | no common name | 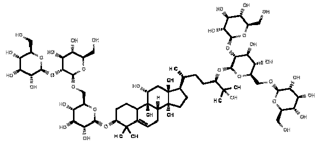 | *Momordica grosvenori* |
| | 1449,588 | | | |
| **7** | C60H102O28 | 11-Deoxyisomogroside V | 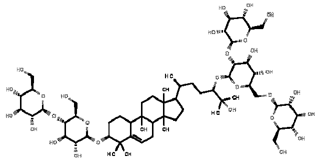 | *Momordica grosvenori* |
| | 1271,448 | | | |
| | 1628293-32-2 | | | |
| **8** | C54H92O23 | no common name | 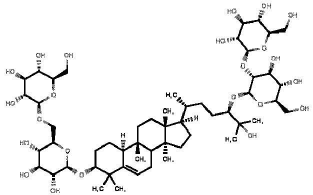 | *Momordica grosvenori* |
| | 1109,307 | | | |
| **9** | C66H112O34 | no common name | 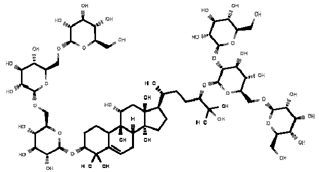 | *Momordica grosvenori* |
| | 1449,588 | | | |
| | 2146088-13-1 | | | |
| **10** | C59H100O28 | no common name | 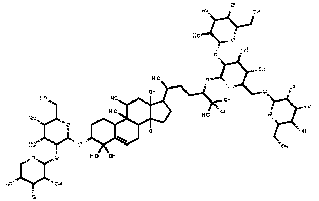 | *Momordica grosvenori* |
| | 1257,421 | | | |
| **11** | C54H90O24 | no common name | 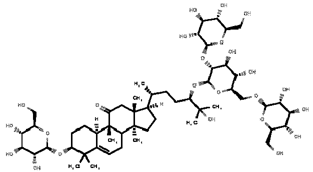 | *Momordica grosvenori* |
| | 1123,29 | | | |
| | 1793003-50-5 | | | |
| **12** | C54H92O23 | no common name | 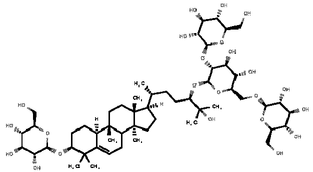 | *Momordica grosvenori* |
| | 1109,307 | | | |
| | 1793003-46-9 | | | |
| **13** | C60H100O29 | 11-Oxoisomogroside V | 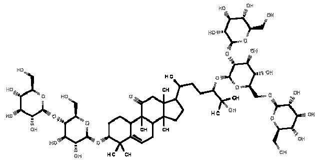 | *Momordica grosvenori* |
| | 1285,431 | | | |
| | 2149606-15-3 | | | |
| **14** | C60H102O29 | no common name | 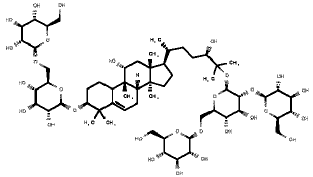 | *Momordica grosvenori* |
| | 1287,447 | | | |
| **15** | C66H110034 | 11-Oxo-mogroside VI | 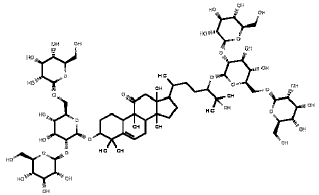 | *Momordica grosvenori* |
| | 1447,572 | | | |
| | 1421942-59-7 | | | |
| **16** | C48H82O20 | no common name | 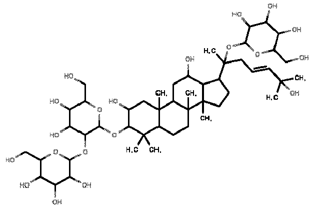 | *Gynostemma pentaphyllum* |
| | 979,164 | | | |
| **17** | C66H112O33 | no common name | 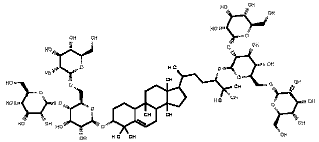 | *Momordica grosvenori* |
| | 1433,589 | | | |
| **18** | C53H90O24 | no common name | 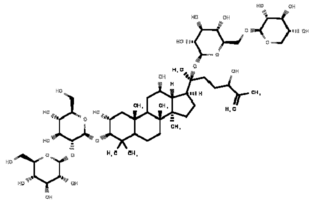 | *Gynostemma pentaphyllum* |
| | 1111,279 | | | |
| **19** | C60H102O29 | no common name | 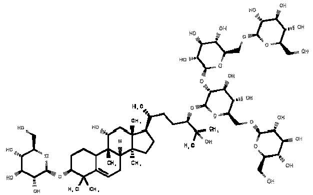 | *Momordica grosvenori* |
| | 1287,447 | | | |
| **20** | C66H112O34 | no common name | 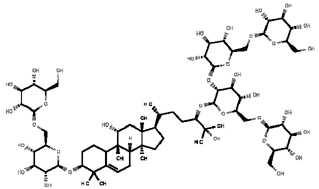 | *Momordica grosvenori* |
| | 1449,588 | | | |
| **21** | C60H102O29 | 11-epi-Mogroside V | 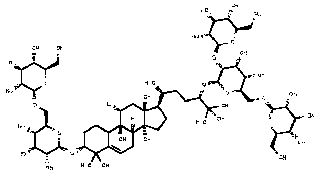 | *Momordica grosvenori* |
| | 1287,447 | | | |
| | 2146088-12-0 | | | |
| **22** | C42H70O14 | no common name | 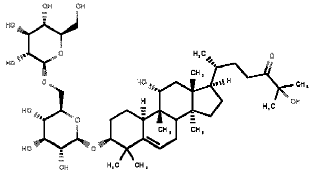 | *Momordica grosvenori* |
| | 799,008 | | | |
| **23** | C48H82O19 | Mogroside III A2 | 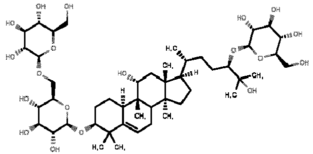 | *Momordica grosvenori* |
| | 963,165 | | | |
| | 88901-43-3 | | | |
| **24** | C42H72O14 | Mogroside II E | 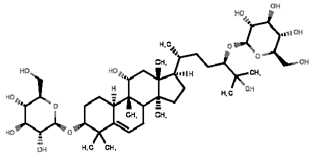 | *Momordica grosvenori* |
| | 801,024 | | | |
| | 88901-38-6 | | | |
| **25** | C48H82O18 | no common name | 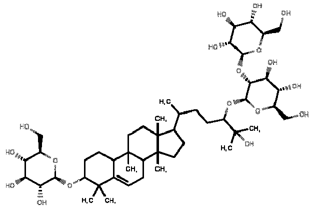 | *Momordica grosvenori* |
| | 947,166 | | | |
| | 1793003-47-0 | | | |
| **26** | C60H100O30 | 7-Oxomogroside V | 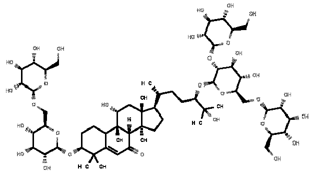 | *Momordica grosvenori* |
| | 1301,43 | | | |
| | 942612-72-8 | | | |
| **27** | C66H112O34 | Neomogroside | 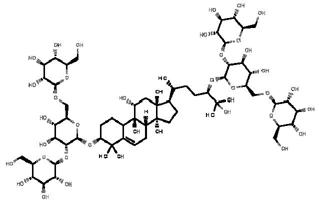 | *Momordica grosvenori* |
| | 1449,588 | | | |
| | 189307-15-1 | | | |
| **28** | C54H92O23 | no common name | 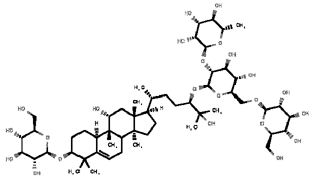 | *Momordica grosvenori* |
| | 1109,307 | | | |
| **29** | C48H80O19 | no common name | 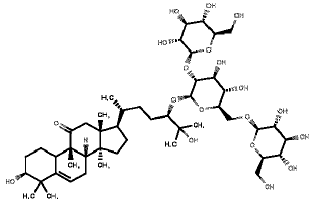 | *Momordica grosvenori* |
| | 961,149 | | | |
| **30** | C48H82O19 | Mogroside III | 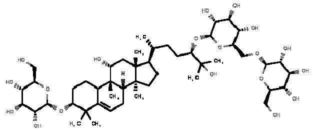 | *Momordica grosvenori* |
| | 963,165 | | | |
| | 130567-83-8 | | | |
| **31** | C54H92O23 | no common name | 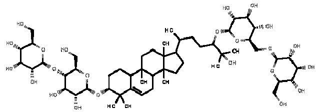 | *Momordica grosvenori* |
| | 1109,307 | | | |
| **32** | C54H92O24 | no common name | 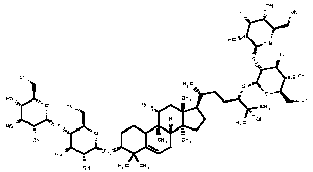 | *Momordica grosvenori* |
| | 1125,306 | | | |
| | 2146088-06-2 | | | |
| **33** | C66H112O34 | no common name | 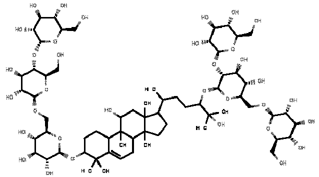 | *Momordica grosvenori* |
| | 1449,588 | | | |
| **34** | C54H92O23 | no common name | 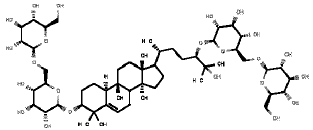 | *Momordica grosvenori* |
| | 1109,307 | | | |
| **35** | C54H90O24 | no common name | 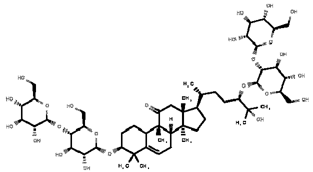 | *Momordica grosvenori* |
| | 1123,29 | | | |
| **36** | C84H142O49 | no common name | 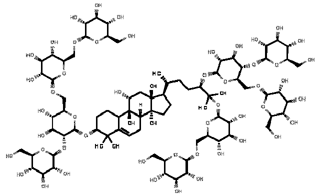 | *Momordica grosvenori* |
| | 1936,011 | | | |
| **37** | C72H122O39 | no common name | 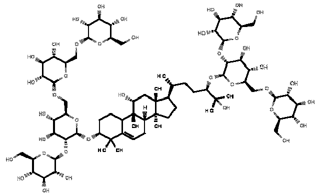 | *Momordica grosvenori* |
| | 1611,729 | | | |
| **38** | C72H122O39 | no common name | 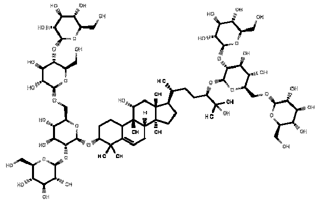 | *Momordica grosvenori* |
| | 1611,729 | | | |
| **39** | C72H122O39 | no common name | 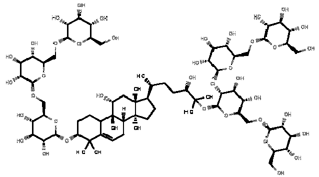 | *Momordica grosvenori* |
| | 1611,729 | | | |
| **40** | C54H90O24 | no common name | 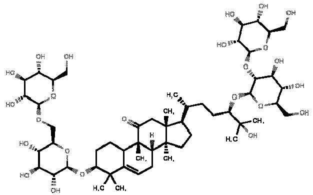 | *Momordica grosvenori* |
| | 1123,29 | | | |
| | 2096516-32-2 | | | |
| **41** | C30H52O6 | Fasciculol C | 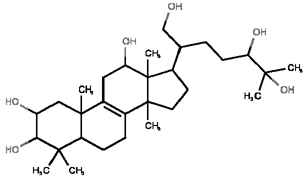 | *Hypholoma fasciculare* |
| | 508,74 | | | |
| | 65694-19-1 | | | |
| **42** | C48H82O18 | Gynosaponin S | 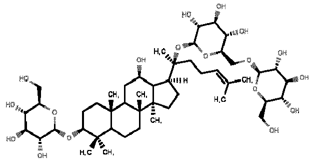 | *Gynostemma pentaphyllum* |
| | 947,166 | | | |
| | 80321-69-3 | | | |
| **43** | C53H90O23 | Gypenoside LVI | 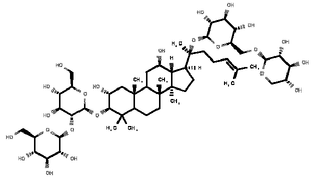 | *Gynostemma pentaphyllum* |
| | 1095,28 | | | |
| | 105214-48-0 | | | |
| **44** | C48H82O19 | Gypenoside XLVI | 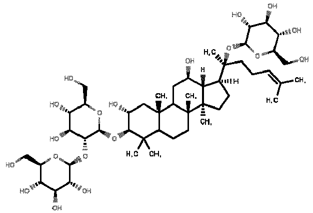 | *Gynostemma pentaphyllum* |
| | 963,165 | | | |
| | 94705-70-1 | | | |
| **45** | C53H90O25 | no common name | 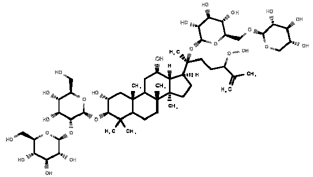 | *Gynostemma pentaphyllum* |
| | 1127,278 | | | |
| | 880874-58-8 | | | |
| **46** | C48H82O21 | Gypenoside GD5 | 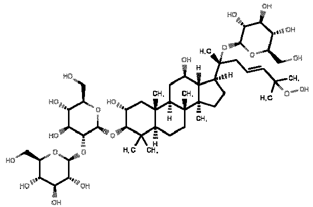 | *Gynostemma pentaphyllum* |
| | 995,163 | | | |
| | 1704440-94-7 | | | |
| **47** | C53H90O23 | no common name | 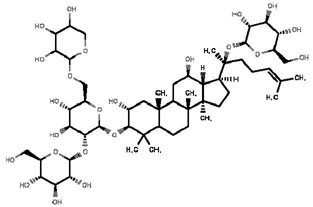 | *Gynostemma pentaphyllum* |
| | 1095,28 | | | |
| **48** | C45H78O20 | no common name | 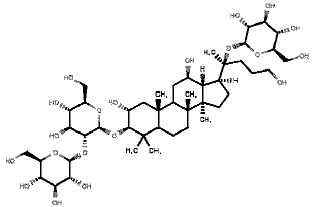 | *Gynostemma pentaphyllum* |
| | 939,099 | | | |
| **49** | C48H82O21 | no common name | 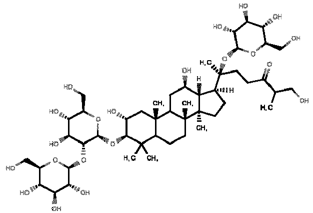 | *Gynostemma pentaphyllum* |
| | 995,163 | | | |
| **50** | C45H76O21 | no common name | 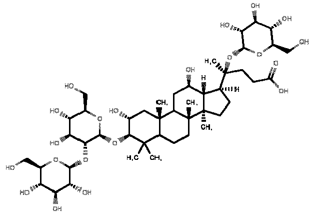 | *Gynostemma pentaphyllum* |
| | 953,082 | | | |
| **51** | C53H90O24 | Gypenoside LXVIII | 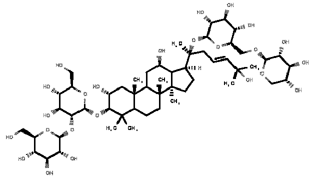 | *Gynostemma pentaphyllum* |
| | 1111,279 | | | |
| | 109145-69-9 | | | |
| **52** | C53H88O24 | no common name | 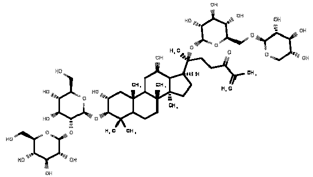 | *Gynostemma pentaphyllum* |
| | 1109,263 | | | |
| **53** | C53H90O25 | no common name | 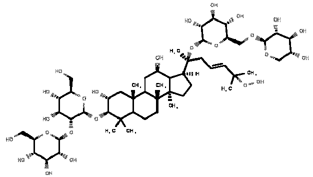 | *Gynostemma pentaphyllum* |
| | 1127,278 | | | |
| | 880874-59-9 | | | |
| **54** | C48H80O20 | no common name | 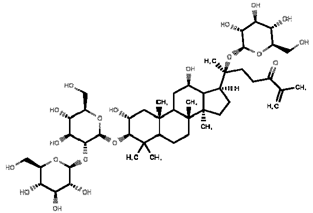 | *Gynostemma pentaphyllum* |
| | 977,148 | | | |
| **55** | C48H80O18 | Bryoside | 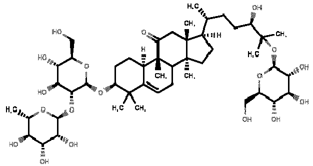 | *Bryonia dioica* |
| | 945,15 | | | |
| | 83725-20-6 | | | |
| **56** | C54H90O23 | Bryonoside | 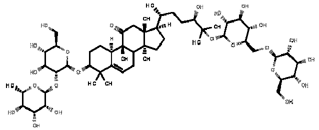 | *Bryonia dioica* |
| | 1107,291 | | | |
| | 83725-21-7 | | | |
| **57** | C41H72O13 | no common name | 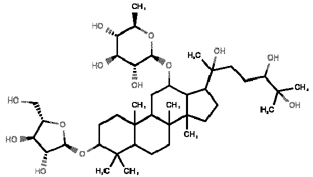 | *Pterocarya paliurus* |
| | 773,014 | | | |
| **58** | C40H68O12 | no common name | 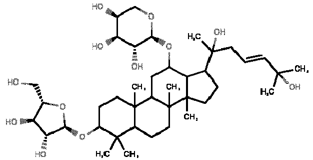 | *Pterocarya paliurus* |
| | 740,972 | | | |
| **59** | C41H70O13 | no common name | 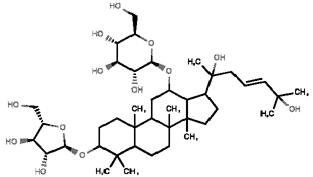 | *Pterocarya paliurus* |
| | 770,998 | | | |
| **60** | C42H66O16 | no common name | 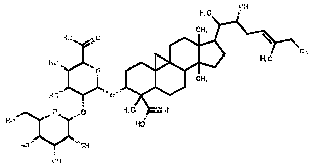 | *Abrus precatorius* |
| | 826,974 | | | |
| **61** | C36H60O9 | no common name | 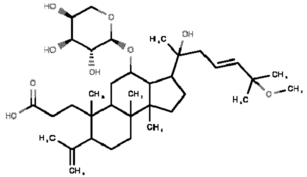 | *Pterocarya paliurus* |
| | 636,867 | | | |
| | 1403937-43-8 | | | |
| **62** | C29H46O8 | no common name | 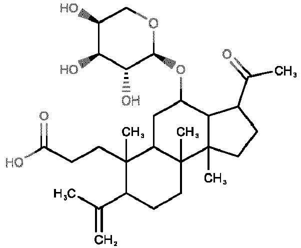 | *Pterocarya paliurus* |
| | 522,679 | | | |
| | 2056021-80-6 | | | |
| **63** | C35H58O9 | no common name | 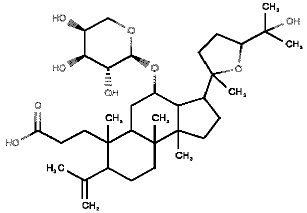 | *Pterocarya paliurus* |
| | 622,84 | | | |
| | 2056021-77-1 | | | |
| **64** | C36H60O9 | Cyclocarioside III | 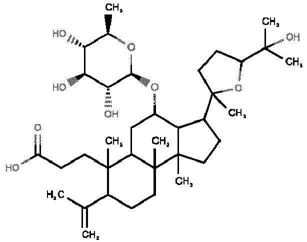 | *Pterocarya paliurus* |
| | 636,867 | | | |
| | 173294-77-4 | | | |
| **65** | C35H58O9 | Cyclocarioside J | 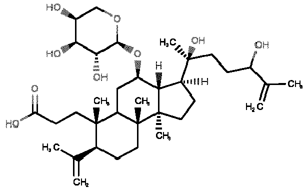 | *Pterocarya paliurus* |
| | 622,84 | | | |
| | 1644624-86-1 | | | |
| **66** | C36H60O10 | no common name | 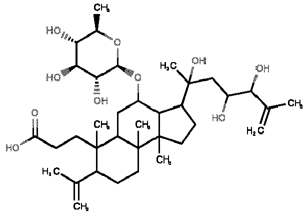 | *Pterocarya paliurus* |
| | 652,866 | | | |
| **67** | C36H60O10 | no common name | 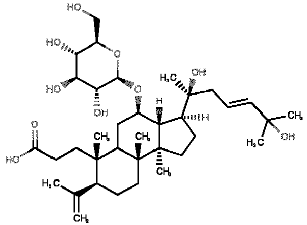 | *Pterocarya paliurus* |
| | 652,866 | | | |
| **68** | C36H60O10 | no common name | 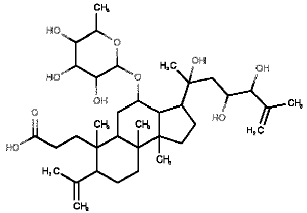 | *Pterocarya paliurus* |
| | 652,866 | | | |
| **69** | C35H58O10 | no common name | 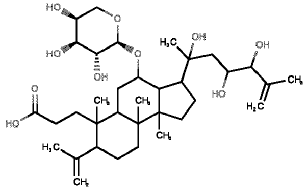 | *Pterocarya paliurus* |
| | 638,839 | | | |
| **70** | C43H72O13 | no common name | 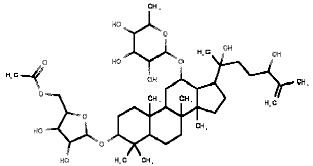 | *Pterocarya paliurus* |
| | 797,036 | | | |
| **71** | C43H72O13 | Cyclocarioside H | 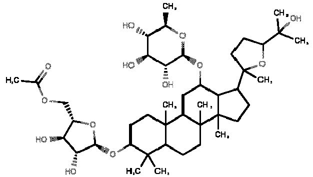 | *Pterocarya paliurus* |
| | 797,036 | | | |
| | 1403937-87-0 | | | |
| **72** | C41H70O12 | Cyclocarioside K | 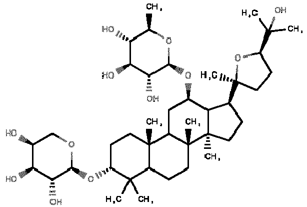 | *Pterocarya paliurus* |
| | 754,999 | | | |
| | 1644624-87-2 | | | |
| **73** | C40H68O12 | no common name | 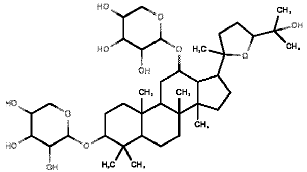 | *Pterocarya paliurus* |
| | 740,972 | | | |
| **74** | C42H72O12 | Cyclocarioside B | 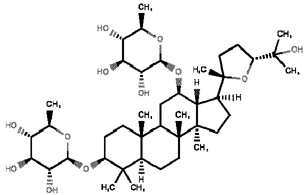 | *Pterocarya paliurus* |
| | 769,026 | | | |
| | 934226-74-1 | | | |
| **75** | C71H114O34 | no common name | 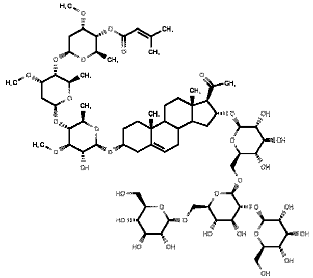 | *Myriopteron extensum* |
| | 1511,659 | | | |
| | 2079912-14-2 | | | |
| **76** | C52H84O27 | no common name | 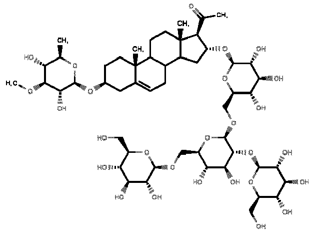 | *Myriopteron extensum* |
| | 1141,217 | | | |
| **77** | C59H94O24 | Extensumside C | 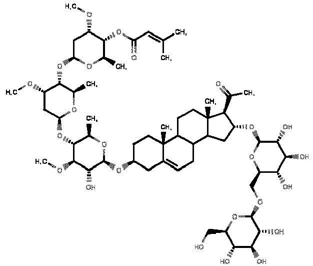 | *Myriopteron extensum* |
| | 1197,377 | | | |
| | 2079912-09-5 | | | |
| **78** | C53H84O22S | no common name | 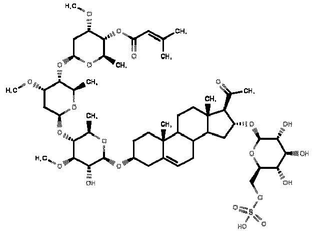 | *Myriopteron extensum* |
| | 1105,29 | | | |
| **79** | C70H112O34 | no common name | 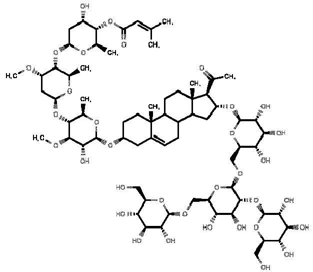 | *Myriopteron extensum* |
| | 1497,632 | | | |
| | 2079912-17-5 | | | |
| **80** | C40H64O17 | no common name | 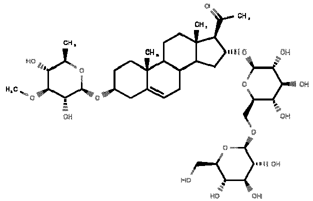 | *Myriopteron extensum* |
| | 816,935 | | | |
| **81** | C46H74O22 | no common name | 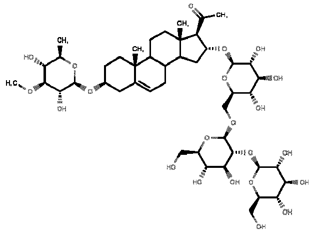 | *Myriopteron extensum* |
| | 979,076 | | | |
| **82** | C46H74O22 | no common name | 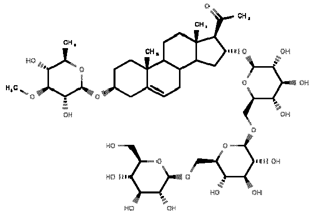 | *Myriopteron extensum* |
| | 979,076 | | | |
| **83** | C65H104O29 | no common name | 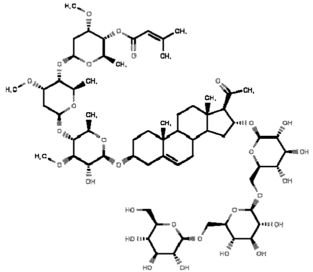 | *Myriopteron extensum* |
| | 1349,518 | | | |
| **84** | C65H104O29 | no common name | 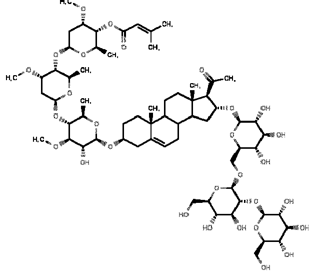 | *Myriopteron extensum* |
| | 1349,518 | | | |
| **85** | C51H84O25 | no common name | 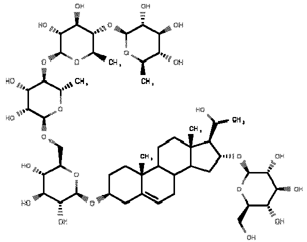 | *Myriopteron extensum* |
| | 1097,208 | | | |
| **86** | C45H74O21 | no common name | 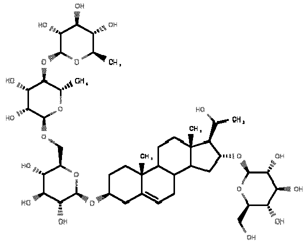 | *Myriopteron extensum* |
| | 951,066 | | | |
| **87** | C53H86O19S | no common name | 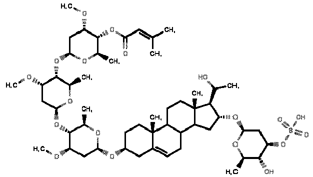 | *Myriopteron extensum* |
| | 1059,31 | | | |
| **88** | C27H42O3 | Diosgenin; (3beta,25R) Spirost-5-en-3-ol | 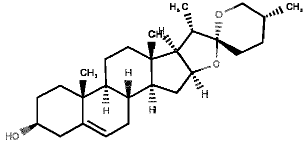 | *Dioscorea sp.* |
| | 414,63 | | | |
| | 512-04-9 | | | |
| **89** | C42H64O15 | Abrusoside C | 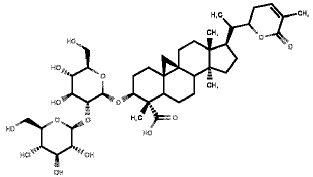 | *Abrus precatorius* |
| | 808,959 | | | |
| | 125002-99-5 | | | |
| **90** | C42H62O16 | Abrusoside E | 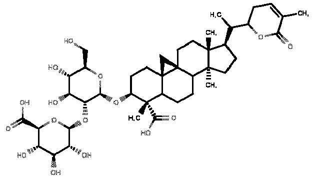 | *Abrus precatorius* |
| | 822,942 | | | |
| | 175448-27-8 | | | |
| **91** | C73H119O38 | Astersaponin C | 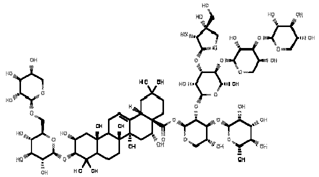 | *Aster tataricus* |
| | 1.603,71 | | | |
| | 125085-17-8 | | | |
| **92** | C42H64O16 | Periandrin III | 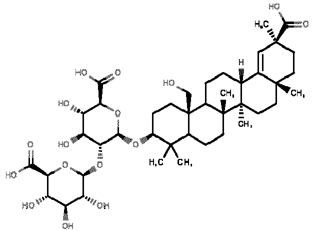 | *Periandra dulcis* |
| | 824,958 | | | |
| | 74256-70-5 | | | |
| **93** | C42H66O16 | Esculentoside S | 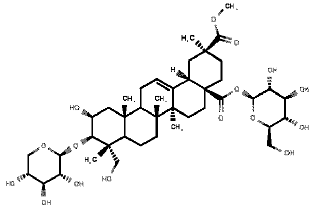 | *Saponaria sp.* |
| | 826,974 | | | |
| | 156031-83-3 | | | |
| **94** | C47H76O18 | Akebiasaponin D | 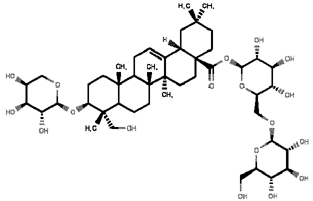 | *Dipsacus sp.* |
| | 929,107 | | | |
| | 39524-08-8 | | | |
| **95** | C63H102O29 | no common name | 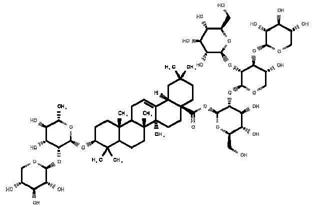 | *Albizia sp.* |
| | 1323,48 | | | |
| **96** | C42H72O14 | no common name | 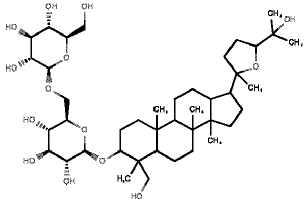 | *Gynostemma pentaphyllum* |
| | 801,024 | | | |
| **97** | C36H60O14 | no common name | 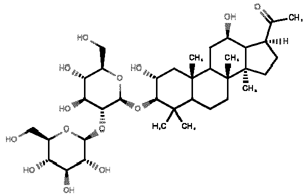 | *Gynostemma pentaphyllum* |
| | 716,862 | | | |
| **98** | C25H40O8 | Gaudichaudioside A | 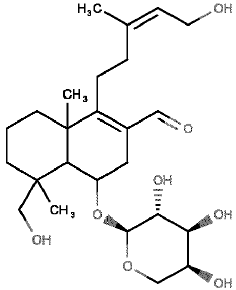 | *Baccharis gaudichaudiana* |
| | 468,587 | | | |
| | 138797-27-0 | | | |
| **99** | C25H44O8 | Gaudichaudioside D | 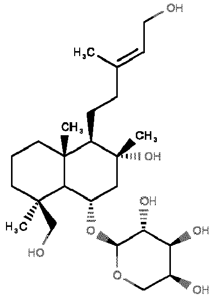 | *Baccharis gaudichaudiana* |
| | 472,619 | | | |
| | 138771-42-3 | | | |
| **100** | C25H42O8 | Gaudichaudioside B | 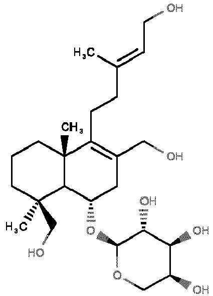 | *Baccharis gaudichaudiana* |
| | 470,603 | | | |
| | 138771-40-1 | | | |
| **101** | C31H48O14S | Phlomisoside II, 6'-sulfate | 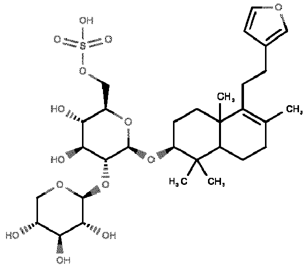 | *Phlomis betonicoides* |
| | 676,77 | | | |
| **102** | C31H48O14S | Phlomisoside II, 3'-sulfate | 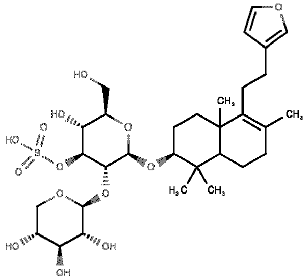 | *Phlomis betonicoides* |
| | 676,77 | | | |
| **103** | C32H50O12 | Phlomisoside II | 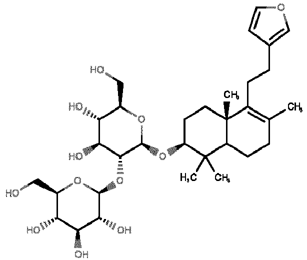 | *Phlomis betonicoides* |
| | 626,74 | | | |
| | 100414-78-6 | | | |
| **104** | C16H14O6 | Hesperetin | 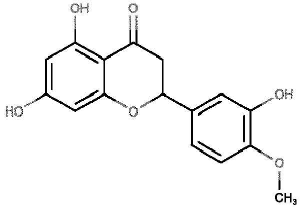 | *Citrus aurantium* |
| | 302,282 ng | | | |
| **105** | C16H14O7 | Padmatin | 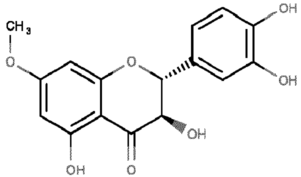 | *Artemisia erithmifolia* |
| | 318,281 | | | |
| | 80453-44-7 | | | |
| **106** | C20H1801O | Kaempferol 3-a-L-arabinoside | 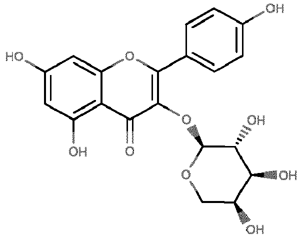 | *Euphorbia paralias. Rubus suavissimus* |
| | 418,354 | | | |
| | 99882-10-7 | | | |
| **107** | C15H12O6 | 3,3',4',7-Tetrahydroxyflavanone | 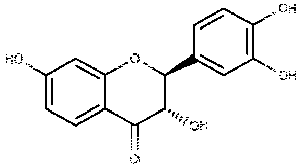 | *Perymenium grande* |
| | 288,255 | | | |
| | 20725-03-5 | | | |
| **108** | C21H22O11 | Neoisoastilbin | 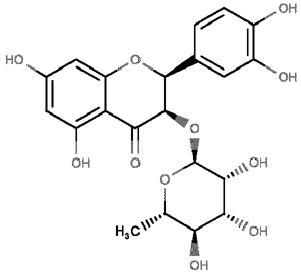 | *Engelhardtia crysolepis* |
| | 450,396 | | | |
| | 54141-72-9 | | | |
| **109** | C45H36O15 | Selligueain A | 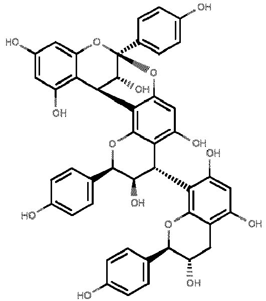 | *Polypodium vulgare* |
| | 816,768 | | | |
| | 152378-18-2 | | | |
| **110** | C21H24O9 | Gnetifolin E | 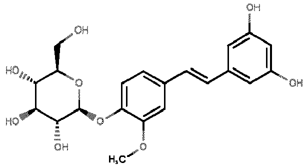 | *Gnetum sp.* |
| | 420,414 | | | |
| | 140671-07-4 | | | |
| **111** | C20H22O9 | Piceatannol 4'-O-β-D-glucopyranoside | 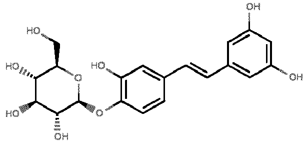 | *Bauhinia cardinalis* |
| | 406,387 | | | |
| | 116181-54-5 | | | |
| **112** | C16H14O6 | Thunberginol E | 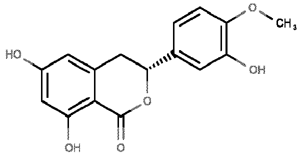 | *Hydrangea macrophylla* |
| | 302,282 | | | |
| | 147517-08-6 | | | |
| **113** | C51H86O28 | no common name | 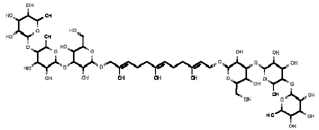 | *Sapindus saponaria* |
| | 1147,221 | | | |
| **114** | C28H57NO8 | no common name | 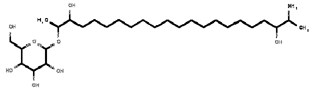 | *Fungi imperfecti* |
| | 535,763 | | | |
| **115** | C9H16O4 | Azelainsäure | 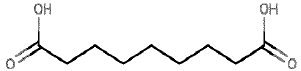 | *Solanum melongena* |
| | 188,223 | | | |
| | 123-99-9 | | | |
| **116** | C18H24O8 | Malaxinic acid | 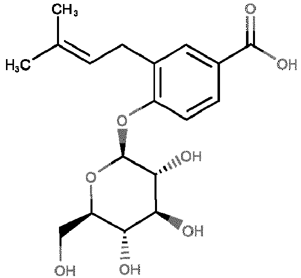 | *Prunus persica* |
| | 368,382 | | | |
| | 23179-40-0 | | | |
| **117** | C28H32O15 | no common name | 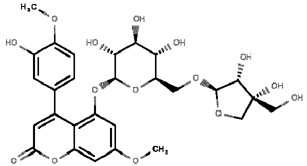 | *Hintonia latiflora* |
| | 608,549 | | | |
| **118** | C19H30O8 | 6,9-Dihydroxy-4,7-megastigmadien-3-one 9-O-β-D-Glucopyranoside | 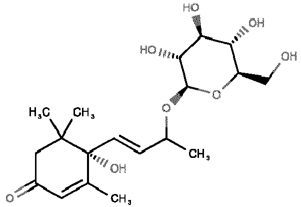 | *Citrus sinensis* |
| | 386,441 | | | |
| **119** | C18H26O10 | 4-[(2,3-Dihydroxy-3-methyl-1-oxobutoxy)methyl]phenyl β-D-glucopyranoside | 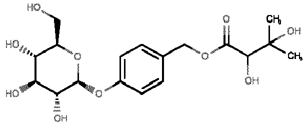 | *Cucurbita pepo* |
| | 402,396 | | | |
| | 321722-02-5 | | | |

### EXAMPLES

The examples which follow are intended to illustrate the present invention without limiting the invention. Unless indicated otherwise all amounts, parts and percentages are based on the weight and the total amount or on the total weight and the total amount of the preparations.

### EXAMPLE 1

### A) Extraction, isolation and identification of compounds of formula 1 to 119

1.0 kg dried plant material was extracted subsequently with 12.51 methanol-MTB-ether (50:50 v/v) and methanol (100%) at room temperature for 24h. Both extracts were combined and the solvent evaporated in vacuum. The extract yield depends on the used plant species and the part of the plant. Typically. The extract yield is about 5 to 25% of the dried plant material.

### B) Pre-fractionation by reverse phase chromatography

The raw extract was subjected to a reverse phase medium pressure chromatography to enrich the compounds of interest. Reverse phase MPLC allows to remove sugars and lipids as well as other secondary metabolites like flavonoids very efficiently and to reach enriched fractions of compounds of interest, which could be put on a second fractions step by HPLC using finer RP material. Sometimes it makes sense, to combine different MPLC prefractionations methods to get a more specific enrichment of selected saponins, e.g. by combination of RP18 and RP4 material.

### C) Second purification step by reverse phase chromatography

Pure compounds were isolated by reverse phase chromatography using different fractions from the first separation step by MPLC. To reach a purity of >90% per compound sometimes several repeated separations by HPLC were necessary. Promising fractions from the preparative HPLC runs detected by light scattering detection ELSD were analyzed by LCMS. The solvent of all fractions containing compounds of interest was removed in vacuum followed by a freeze drying step. Isolated compounds were characterized by LCMS and 1D and 2D NMR spectroscopy.

### EXAMPLE 2

### Organoleptic test of the compounds against sucrose

The isolated pure compounds were dissolved in non-carbonated mineral water ("Evian") in a concentration of 0.4 mg/ml (400 ppm). The sweet taste of each sample was compared by a panel of 3-4 panelists with a solution of sucrose in a concentration of 20 g/l.

The sweetness was evaluated as follows:
3 = sweeter than the control solution
2 = sweetness comparable with the control solution
1 = less sweet than the control solution but still sweet

### EXAMPLE 3

### Organoleptic test of the compounds in combination with sucrose against sucrose (Sucrose enhancement)

The isolated pure compounds were dissolved in non-carbonated mineral water ("Evian") in a concentration of 50 ppm (0.05 mg/ml), 25 ppm (0.025 mg/ml), 12.5 pm (0.0125 mg/ml) and 6.125 ppm (0.00625 mg/ml). The sweet taste of each sample was detected by a panel of 3-4 panelists to detect the sweetness perception level. The highest concentration below the sweetness perception level was used for the evaluation of the sweet taste enhancement of sucrose.

A solution of the isolated compound in a concentration below the sweetness perception level was dissolved in 6% sucrose solution. The sweet taste of this sample was compared to the sweet taste of a 6% sucrose solution by a panel of 12 to 15 panelists in a two-alternative forced-choice test (2-AFC. E2164-16_Standard Test Method for Directional Difference Test). The panelists were trained to differentiate between the sweetness of a 6% and a 7% Sucrose solution.

### EXAMPLE 4

### Organoleptic test of the compounds in combination with sucrose against Rebaudioside M (Rebaudioside M enhancement)

The isolated pure compounds were dissolved in non-carbonated mineral water ("Evian") in a concentration of 50 ppm (0.05 mg/ml), 25 ppm (0.025 mg/ml), 12.5 pm (0.0125 mg/ml) and 6.125 ppm (0.006125 mg/ml). The sweet taste of each sample was detected by a panel of 3-4 panelists to detect the sweetness perception level. The highest concentration below the sweetness perception level was used for the evaluation of the sweet taste enhancement of Rebaudioside M.

A solution of the isolated compound in a concentration below the sweetness perception level was dissolved in 150 ppm Rebaudioside M solution. The sweet taste of this sample was compared to the sweet taste of a 150 ppm Rebaudioside M solution by a panel of 12 to 15 panelists in a two-alternative forced-choice test (2-AFC. E2164-16_Standard Test Method for Directional Difference Test). The panelists were trained to differentiate between the sweetness of a 150 ppm and a 190 ppm Rebaudioside M solution. Results are listed in **Table 1.**

**Table 1**

| Organoleptic evaluation | | | |
|---|---|---|---|
| **Compound #** | **Sweetness** | **Sucrose enhancement x = detected** | **Reb M enhancement x = detected** |
| 1 | 2 | x | |
| 2 | 1 | x | |
| 3 | 3 | | x |
| 4 | 2 | | x |
| 5 | 2 | x | |
| 6 | 1 | | |
| 7 | 1 | | x |
| 8 | 3 | | |
| 9 | 3 | | |
| 10 | 2 | | |
| 11 | 1 | | x |
| 12 | 2 | | x |
| 13 | 1 | | |
| 14 | 3 | | |
| 15 | 3 | x | |
| 16 | 2 | | x |
| 17 | 2 | | |
| 18 | 1 | | x |
| 19 | 1 | | |
| 20 | 3 | | |
| 21 | 1 | x | |
| 22 | 3 | | x |
| 23 | 1 | x | |
| 24 | 2 | x | |
| 25 | 2 | x | |
| 26 | 2 | x | |
| 27 | 2 | x | |
| 28 | 3 | | |
| 29 | 3 | | |
| 30 | 2 | x | |
| 31 | 3 | | |
| 32 | 3 | | |
| 33 | 3 | | |
| 34 | 3 | | |
| 35 | 3 | | |
| 36 | 2 | | |
| 37 | 3 | | |
| 38 | 3 | | |
| 39 | 3 | | |
| 40 | 3 | | |
| 41 | 3 | x | x |
| 42 | 1 | | |
| 43 | 1 | | x |
| 44 | 1 | | x |
| 45 | 3 | | x |
| 46 | 2 | | |
| 47 | 2 | | |
| 48 | 3 | | |
| 49 | 3 | | |
| 50 | 3 | | |
| 51 | 3 | | x |
| 52 | 1 | | |
| 53 | 1 | | x |
| 54 | 1 | | |
| 55 | 0 | | x |
| 56 | 0 | | x |
| 57 | 0 | x | |
| 58 | 2 | | |
| 59 | 0 | x | |
| 60 | 0 | x | |
| 61 | 1 | x | |
| 62 | 0 | | x |
| 63 | 3 | | x |
| 64 | 2 | x | |
| 65 | 3 | | |
| 66 | 2 | | |
| 67 | 2 | | |
| 68 | 3 | | |
| 69 | 1 | | |
| 70 | 1 | | |
| 71 | 1 | | |
| 72 | 3 | | x |
| 73 | 3 | | |
| 74 | 3 | x | |
| 75 | 3 | x | |
| 76 | 3 | | |
| 77 | 3 | x | |
| 78 | 2 | | |
| 79 | 1 | x | |
| 80 | 3 | | |
| 81 | 2 | | |
| 82 | 3 | | |
| 83 | 2 | x | |
| 84 | 2 | x | |
| 85 | 3 | | |
| 86 | 3 | | |
| 87 | 2 | | |
| 88 | 2 | | |
| 89 | 2 | | x |
| 90 | 2 | | x |
| 91 | 1 | x | x |
| 92 | 1 | x | |
| 93 | 1 | | |
| 94 | 3 | x | |
| 95 | 3 | | |
| 96 | 1 | | |
| 97 | 2 | | |
| 98 | 3 | | x |
| 99 | 3 | | x |
| 100 | 1 | | x |
| 101 | 1 | | x |
| 102 | 3 | | x |
| 103 | 3 | x | |
| 104 | 0 | x | x |
| 105 | 3 | x | |
| 106 | 3 | | |
| 107 | 1 | x | |
| 108 | 1 | | |
| 109 | 1 | x | |
| 110 | 1 | | x |
| 111 | 0 | | x |
| 112 | 0 | x | |
| 113 | 0 | x | |
| 114 | 0 | x | |
| 115 | 0 | x | |
| 116 | 1 | x | |
| 117 | 1 | | |
| 118 | 1 | x | |
| 119 | 3 | x | |

| | | | |
|---|---|---|---|
| ¹ Solution of the compound at 400 ppm compared to sucrose solution: 0 no intrinsic sweetness; 1: less sweet than 2% sucrose; isosweet to 2% sucrose; 3: sweeter than 2% sucrose | | | |

### FORMULATION EXAMPLES

The following **Tables 2a to 2f** provide some examples for oral compositions comprising at least one of the sweeteners disclosed before.

**Table 2a**

| Chewing gum, free of sugar; all amounts in % b.w. | | | |
|---|---|---|---|
| **Composition** | **I** | **II** | **III** |
| Gum base | 30.00 | 30.00 | 30.00 |
| Sorbitol, powdered | 40.00 | 40.00 | 40.00 |
| Isomalt, powdered | 9.50 | 9.50 | 9.50 |
| Xylitol | 2.00 | 2.00 | 2.00 |
| Mannitol D | 3.00 | 3.00 | 3.00 |
| Compound 2 | 0.10 | - | - |
| Compound 5 | - | 0.01 | - |
| Compound 7 | - | - | 0.05 |
| Emulgum / Plasticizing agent | 0.30 | 0.30 | 0.30 |
| Sorbitol (70% water) | 13.00 | 13.00 | 13.00 |
| Spearmint aroma | 1.00 | 1.00 | 1.00 |
| Glycerol | Ad 100 | | |

**Table 2b**

| Tooth paste; all amounts in % b.w. | | | |
|---|---|---|---|
| **Composition** | **IV** | **V** | **VI** |
| Glycerol | 20.00 | 20.00 | 20.00 |
| Solbrol M (sodium salt) | 0.15 | 0.15 | 0.15 |
| Sodium monofluor phosphate | 0.76 | 0.76 | 0.76 |
| Compound 41 | 0.20 | - | - |
| Compound 92 | - | 0.10 | - |
| Compound 5 | - | - | 0.01 |
| Dicalciumphosphate dihydrate | 36.00 | 36.00 | 36.00 |
| Aerosil 200 | 3.00 | 3.00 | 3.00 |
| Sodium carboxymethyl cellulose | 1.20 | 1.20 | 1.20 |
| Sodium lauryl sulfate | 1.30 | 1.30 | 1.30 |
| Peppermint aroma | 1.00 | 1.00 | 1.00 |
| Deionised water | Ad 100 | | |

**Table 2c**

| Mouth wash concentrate; all amounts in % b.w. | | | |
|---|---|---|---|
| **Composition** | **VII** | **VIII** | **IX** |
| Ethanol 96% | 42.00 | 42.00 | 42.00 |
| Cremophor RH 455 | 5.00 | 5.00 | 5.00 |
| Allantoin | 0.20 | 0.20 | 0.20 |
| Compound 116 | 0.10 | - | - |
| Compound 117 | - | 0.01 | - |
| Colour L-Blue 5000 (1% in Wasser) | 0.03 | 0.03 | 0.03 |
| Spearmint aroma | 2.00 | 2.00 | 2.00 |
| Deionised water | Ad 100 | | |

**Table 2d**

| Hard boiled candy, sugar-free; all amounts in % b.w. | | | |
|---|---|---|---|
| **Composition** | **X** | **XI** | **XII** |
| Isomalt | 94.98 | 94.98 | 94.98 |
| Xylitol | 2.40 | 2.40 | 2.40 |
| Compound 16 | 0.10 | - | - |
| Compound 18 | - | 0.01 | - |
| Citric acid | 0.050 | 0.050 | 0.050 |
| Cherry aroma | 0.25 | 0.25 | 0.25 |
| Water | Ad 100 | | |

**Table 2e**

| Ice tea | | | |
|---|---|---|---|
| **Composition** | **XIII** | **XIV** | **XV** |
| Sucrose | 3.75 | 3.75 | 1.25 |
| BlackTea Powder | 0.25 | 0.25 | 0.25 |
| Citric Acid | 0.09 | 0.09 | 0.09 |
| Potassium Sorbate | 0.015 | 0.015 | 0.015 |
| Peach Flavor | 0.03 | 0.03 | 0.03 |
| Compound 11 | - | 0.04 | 0.02 |
| Compound 12 | 0.05 | 0.04 | - |
| Rebaudioside A | - | - | 0.01 |
| Water | Ad 100 | | |

**Table 2f**

| Carbonated soft drink | | | |
|---|---|---|---|
| **Composition** | **XVI** | **XVII** | **XVIII** |
| Sucrose | 3.0 | 3.0 | 1.0 |
| Cola Flavor | 0.4 | 0.4 | 0.4 |
| Phosphoric acid 85% | 0.03 | 0.03 | 0.03 |
| Caffeine | 0.01 | 0.01 | 0.01 |
| Compound 89 | 0.05 | 0.02 | |
| Compound 90 | | 0.02 | 0.02 |
| Acesulfam K | | | 0.01 |
| Carbonated water | | Ad 100 | |

## Claims

1. A sweetener or sweetness enhancer composition comprising, consisting or essentially consisting of at least one of Compounds 1 to 119 as defined in the following:
| **C** | **Formula MW Cas-No.** | **Name** | **Structure** | **Biomaterial Source** |
|---|---|---|---|---|
| **1** | C48H82O19 | Mogroside III E | 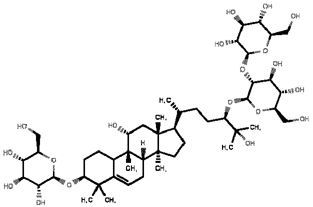 | *Momordica grosvenori* |
| | 963,165 | | | |
| | 88901-37-5 | | | |
| **2** | C48H82O19 | Mogroside III A1 | 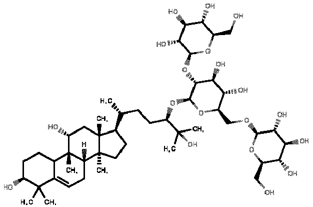 | *Momordica grosvenori* |
| | 963,165 | | | |
| | 88901-42-2 | | | |
| **3** | C42H72O14 | Mogroside II A | 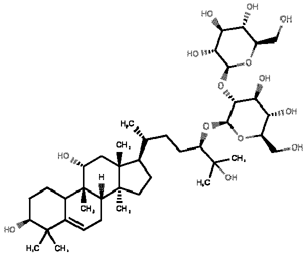 | *Momordica grosvenori* |
| | 801,024 | | | |
| | 1613527-65-3 | | | |
| **4** | C60H102O28 | no common name | 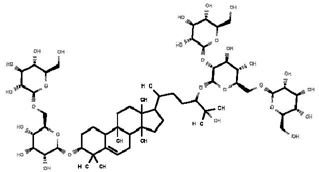 | *Momordica grosvenori* |
| | 1271,448 | | | |
| | 1707161-17-8 | | | |
| **5** | C60H102O29 | Isomogroside V | 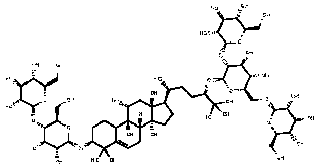 | *Momordica grosvenori* |
| | 1287,447 | | | |
| | 1126032-65-2 | | | |
| **6** | C66H112O34 | no common name | 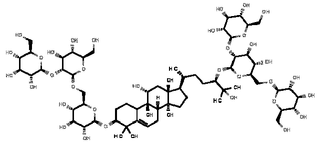 | *Momordica grosvenori* |
| | 1449,588 | | | |
| **7** | C60H102O28 | 11-Deoxyisomogroside V | 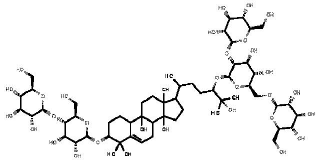 | *Momordica grosvenori* |
| | 1271,448 | | | |
| | 1628293-32-2 | | | |
| **8** | C54H92O23 | no common name | 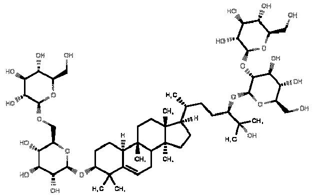 | *Momordica grosvenori* |
| | 1109,307 | | | |
| **9** | C66H112O34 | no common name | 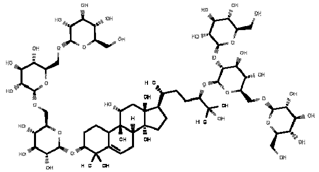 | *Momordica grosvenori* |
| | 1449,588 | | | |
| | 2146088-13-1 | | | |
| **10** | C59H100O28 | no common name | 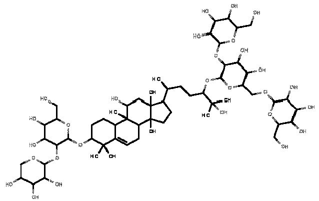 | *Momordica grosvenori* |
| | 1257,421 | | | |
| **11** | C54H90O24 | no common name | 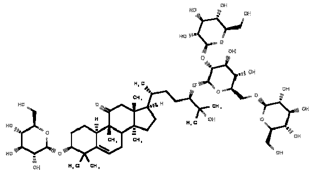 | *Momordica grosvenori* |
| | 1123,29 | | | |
| | 1793003-50-5 | | | |
| **12** | C54H92O23 | no common name | 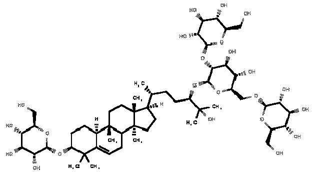 | *Momordica grosvenori* |
| | 1109,307 | | | |
| | 1793003-46-9 | | | |
| **13** | C60H100O29 | 11-Oxoisomogroside V | 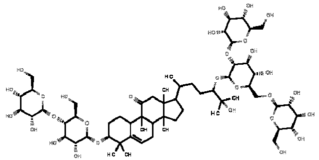 | *Momordica grosvenori* |
| | 1285,431 | | | |
| | 2149606-15-3 | | | |
| **14** | C60H102O29 | no common name | 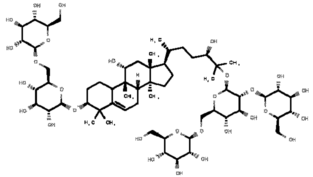 | *Momordica grosvenori* |
| | 1287,447 | | | |
| **15** | C66H110O34 | 11-oxo-mogroside VI | 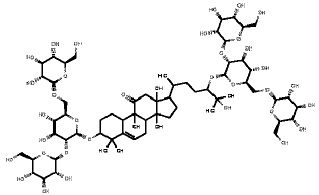 | *Momordica grosvenori* |
| | 1447,572 | | | |
| | 1421942-59-7 | | | |
| **16** | C48H82O20 | no common name | 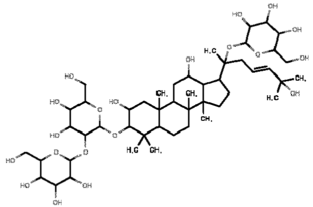 | *Gynostemma pentaphyllum* |
| | 979,164 | | | |
| **17** | C66H112O33 | no common name | 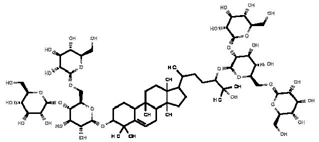 | *Momordica grosvenori* |
| | 1433,589 | | | |
| **18** | C53H90O24 | no common name | 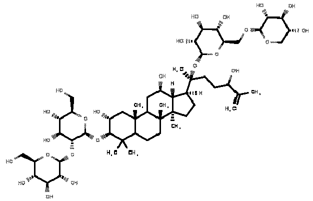 | *Gynostemma pentaphyllum* |
| | 1111,279 | | | |
| **19** | C60H102O29 | no common name | 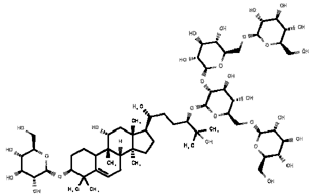 | *Momordica grosvenori* |
| | 1287,447 | | | |
| **20** | C66H112O34 | no common name | 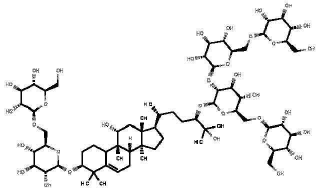 | *Momordica grosvenori* |
| | 1449,588 | | | |
| **21** | C60H102O29 | 11-epi-Mogroside V | 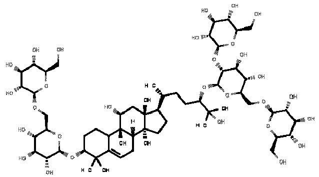 | *Momordica grosvenori* |
| | 1287,447 | | | |
| | 2146088-12-0 | | | |
| **22** | C42H70O14 | no common name | 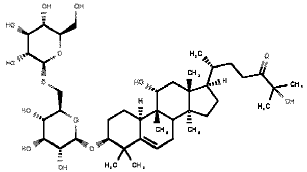 | *Momordica grosvenori* |
| | 799,008 | | | |
| **23** | C48H82O19 | Mogroside III A2 | 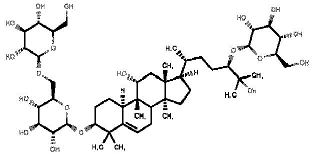 | *Momordica grosvenori* |
| | 963,165 | | | |
| | 88901-43-3 | | | |
| **24** | C42H72O14 | Mogroside II E | 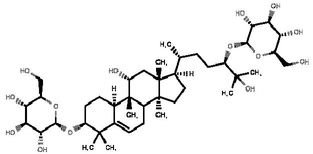 | *Momordica grosvenori* |
| | 801,024 | | | |
| | 88901-38-6 | | | |
| **25** | C48H82O18 | no common name | 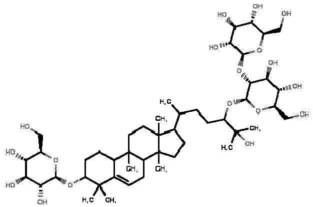 | *Momordica grosvenori* |
| | 947,166 | | | |
| | 1793003-47-0 | | | |
| **26** | C60H100O30 | 7-Oxomogroside V | 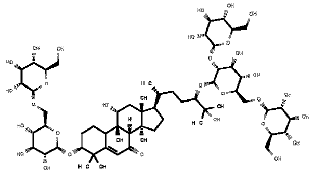 | *Momordica grosvenori* |
| | 1301,43 | | | |
| | 942612-72-8 | | | |
| **27** | C66H112O34 | Neomogroside | 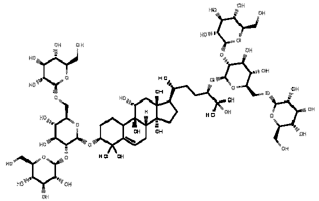 | *Momordica grosvenori* |
| | 1449,588 | | | |
| | 189307-15-1 | | | |
| **28** | C54H92O23 | no common name | 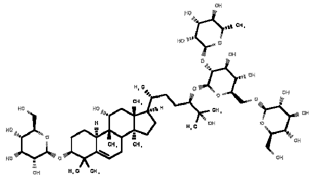 | *Momordica grosvenori* |
| | 1109,307 | | | |
| **29** | C48H80O19 | no common name | 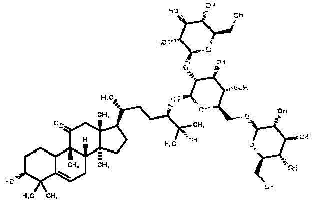 | *Momordica grosvenori* |
| | 961,149 | | | |
| **30** | C48H82O19 | Mogroside III | 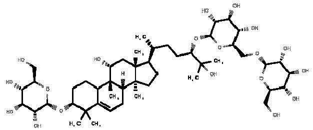 | *Momordica grosvenori* |
| | 963,165 | | | |
| | 130567-83-8 | | | |
| **31** | C54H92O23 | no common name | 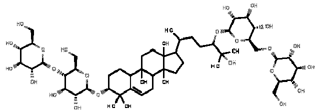 | *Momordica grosvenori* |
| | 1109,307 | | | |
| **32** | C54H92O24 | no common name | 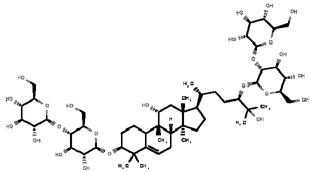 | *Momordica grosvenori* |
| | 1125,306 | | | |
| | 2146088-06-2 | | | |
| **33** | C66H112O34 | no common name | 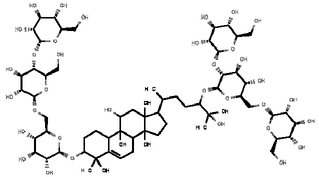 | *Momordica grosvenori* |
| | 1449,588 | | | |
| **34** | C54H92O23 | no common name | 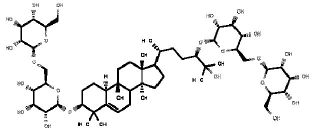 | *Momordica grosvenori* |
| | 1109,307 | | | |
| **35** | C54H90O24 | no common name | 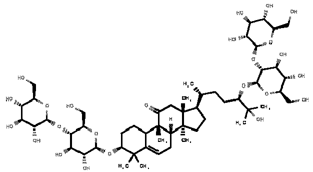 | *Momordica grosvenori* |
| | 1123,29 | | | |
| **36** | C84H142O49 | no common name | 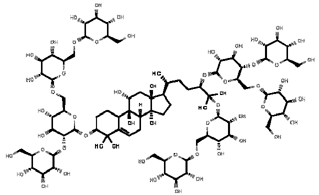 | *Momordica grosvenori* |
| | 1936,011 | | | |
| **37** | C72H122O39 | no common name | 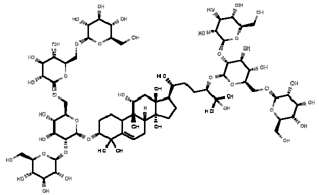 | *Momordica grosvenori* |
| | 1611,729 | | | |
| **38** | C72H122O39 | no common name | 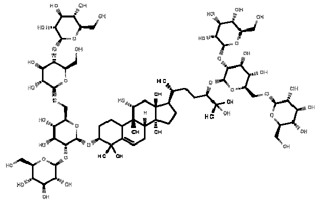 | *Momordica grosvenori* |
| | 1611,729 | | | |
| **39** | C72H122O39 | no common name | 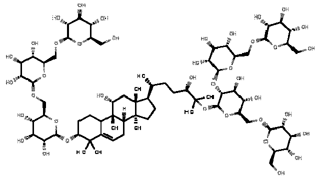 | *Momordica grosvenori* |
| | 1611,729 | | | |
| **40** | C54H90O24 | no common name | 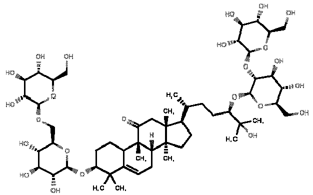 | *Momordica grosvenori* |
| | 1123,29 | | | |
| | 2096516-32-2 | | | |
| **41** | C30H52O6 | Fasciculol C | 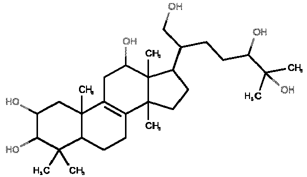 | *Hypholoma fasciculare* |
| | 508,74 | | | |
| | 65694-19-1 | | | |
| **42** | C48H82O18 | Gynosaponin S | 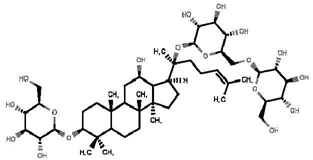 | *Gynostemma pentaphyllum* |
| | 947,166 | | | |
| | 80321-69-3 | | | |
| **43** | C53H90O23 | Gypenoside LVI | 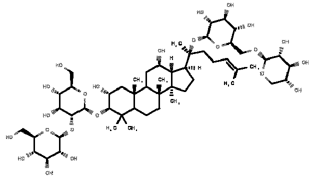 | *Gynostemma pentaphyllum* |
| | 1095,28 | | | |
| | 105214-48-0 | | | |
| **44** | C48H82O19 | Gypenoside XLVI | 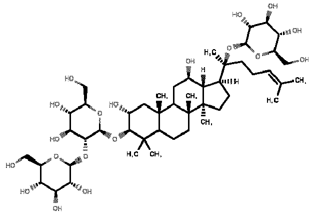 | *Gynostemma pentaphyllum* |
| | 963,165 | | | |
| | 94705-70-1 | | | |
| **45** | C53H90O25 | no common name | 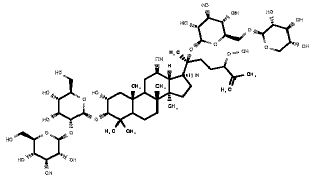 | *Gynostemma pentaphyllum* |
| | 1127,278 | | | |
| | 880874-58-8 | | | |
| **46** | C48H82O21 | Gypenoside GD5 | 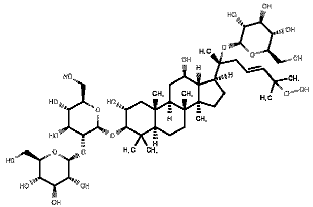 | *Gynostemma pentaphyllum* |
| | 995,163 | | | |
| | 1704440-94-7 | | | |
| **47** | C53H90O23 | no common name | 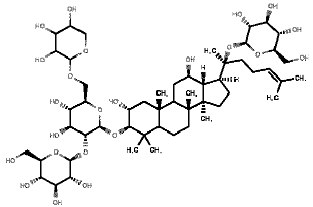 | *Gynostemma pentaphyllum* |
| | 1095,28 | | | |
| **48** | C45H78O20 | no common name | 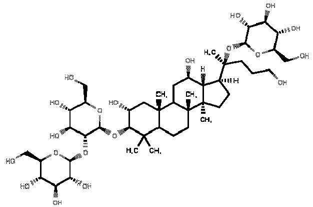 | *Gynostemma pentaphyllum* |
| | 939,099 | | | |
| **49** | C48H82O21 | no common name | 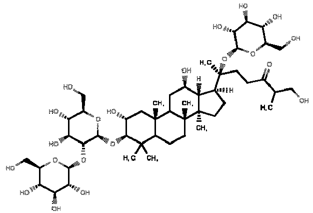 | *Gynostemma pentaphyllum* |
| | 995,163 | | | |
| **50** | C45H76O21 | no common name | 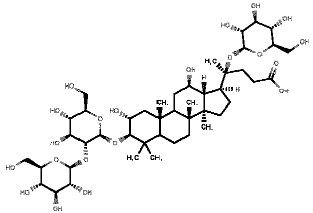 | *Gynostemma pentaphyllum* |
| | 953,082 | | | |
| **51** | C53H90O24 | Gypenoside LXVIII | 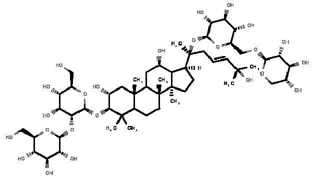 | *Gynostemma pentaphyllum* |
| | 1111,279 | | | |
| | 109145-69-9 | | | |
| **52** | C53H88O24 | no common name | 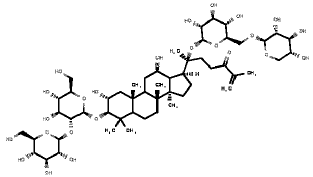 | *Gynostemma pentaphyllum* |
| | 1109,263 | | | |
| **53** | C53H90O25 | no common name | 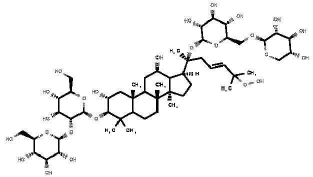 | *Gynostemma pentaphyllum* |
| | 1127,278 | | | |
| | 880874-59-9 | | | |
| **54** | C48H80O20 | no common name | 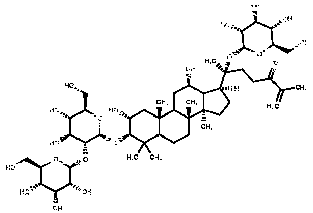 | *Gynostemma pentaphyllum* |
| | 977,148 | | | |
| **55** | C48H80O18 | Bryoside | 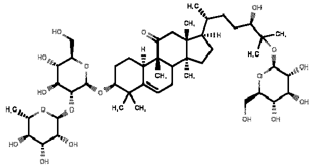 | *Bryonia dioica* |
| | 945,15 | | | |
| | 83725-20-6 | | | |
| **56** | C54H90O23 | Bryonoside | 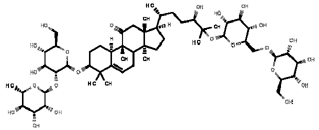 | *Bryonia dioica* |
| | 1107,291 | | | |
| | 83725-21-7 | | | |
| **57** | C41H72O13 | no common name | 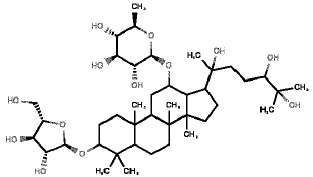 | *Pterocarya paliurus* |
| | 773,014 | | | |
| **58** | C40H68O12 | no common name | 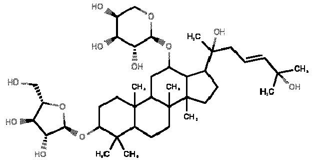 | *Pterocarya paliurus* |
| | 740,972 | | | |
| **59** | C41H70O13 | no common name | 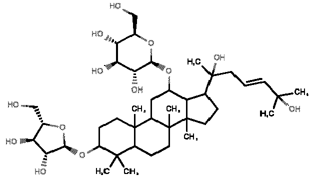 | *Pterocarya paliurus* |
| | 770,998 | | | |
| **60** | C42H66O16 | no common name | 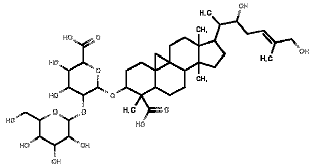 | *Abrus precatorius* |
| | 826,974 | | | |
| **61** | C36H60O9 | no common name | 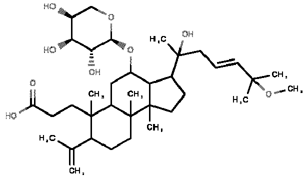 | *Pterocarya paliurus* |
| | 636,867 | | | |
| | 1403937-43-8 | | | |
| **62** | C29H46O8 | no common name | 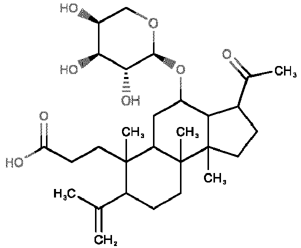 | *Pterocarya paliurus* |
| | 522,679 | | | |
| | 2056021-80-6 | | | |
| **63** | C35H58O9 | no common name | 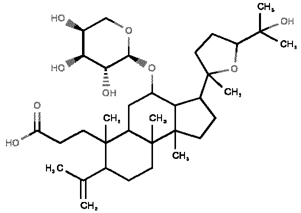 | *Pterocarya paliurus* |
| | 622,84 | | | |
| | 2056021-77-1 | | | |
| **64** | C36H60O9 | Cyclocarioside III | 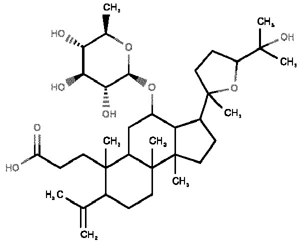 | *Pterocarya paliurus* |
| | 636,867 | | | |
| | 173294-77-4 | | | |
| **65** | C35H58O9 | Cyclocarioside J | 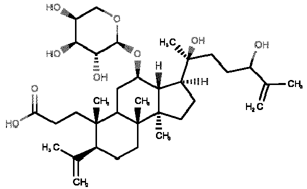 | *Pterocarya paliurus* |
| | 622,84 | | | |
| | 1644624-86-1 | | | |
| **66** | C36H60O10 | no common name | 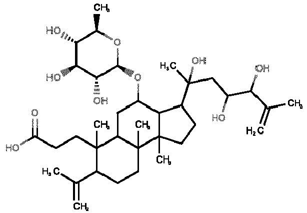 | *Pterocarya paliurus* |
| | 652,866 | | | |
| **67** | C36H60O10 | no common name | 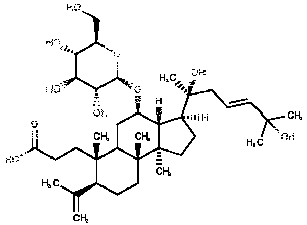 | *Pterocarya paliurus* |
| | 652,866 | | | |
| **68** | C36H60O10 | no common name | 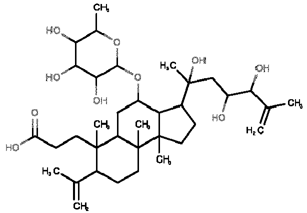 | *Pterocarya paliurus* |
| | 652,866 | | | |
| **69** | C35H58O10 | no common name | 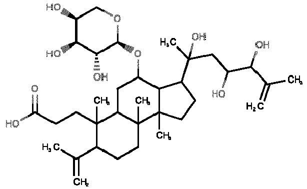 | *Pterocarya paliurus* |
| | 638,839 | | | |
| **70** | C43H72O13 | no common name | 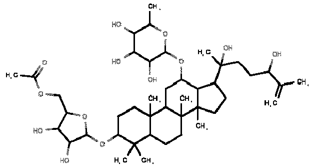 | *Pterocarya paliurus* |
| | 797,036 | | | |
| **71** | C43H72O13 | Cyclocarioside H | 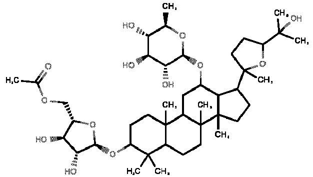 | *Pterocarya paliurus* |
| | 797,036 | | | |
| | 1403937-87-0 | | | |
| **72** | C41H70O12 | Cyclocarioside K | 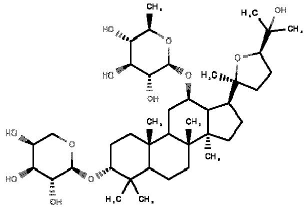 | *Pterocarya paliurus* |
| | 754,999 | | | |
| | 1644624-87-2 | | | |
| **73** | C40H68O12 | no common name | 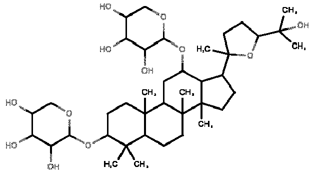 | *Pterocarya paliurus* |
| | 740,972 | | | |
| **74** | C42H72O12 | Cyclocarioside B | 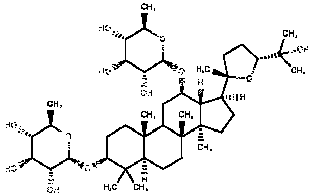 | *Pterocarya paliurus* |
| | 769,026 | | | |
| | 934226-74-1 | | | |
| **75** | C71H114O34 | no common name | 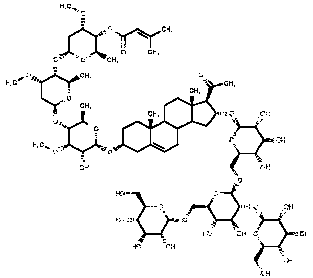 | *Myriopteron extensum* |
| | 1511,659 | | | |
| | 2079912-14-2 | | | |
| **76** | C52H84O27 | no common name | 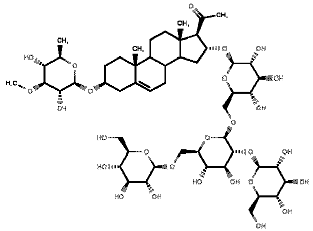 | *Myriopteron extensum* |
| | 1141,217 | | | |
| **77** | C59H94O24 | Extensumside C | 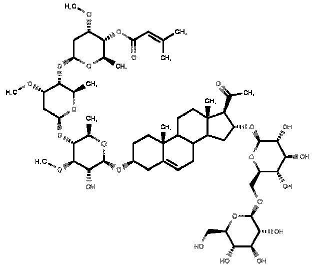 | *Myriopteron extensum* |
| | 1197,377 | | | |
| | 2079912-09-5 | | | |
| **78** | C53H84O22S | no common name | 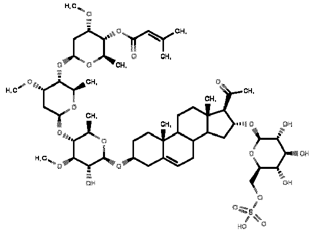 | *Myriopteron extensum* |
| | 1105,29 | | | |
| **79** | C70H112O34 | no common name | 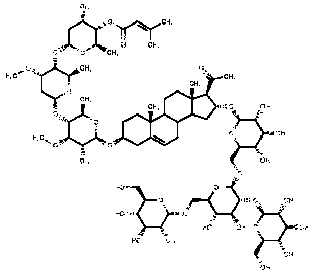 | *Myriopteron extensum* |
| | 1497,632 | | | |
| | 2079912-17-5 | | | |
| **80** | C40H64O17 | no common name | 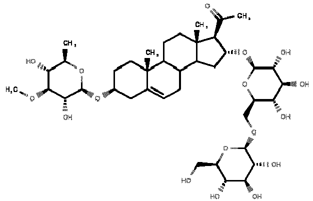 | *Myriopteron extensum* |
| | 816,935 | | | |
| **81** | C46H74O22 | no common name | 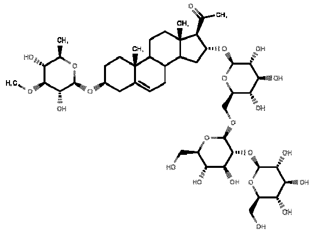 | *Myriopteron extensum* |
| | 979,076 | | | |
| **82** | C46H74O22 | no common name | 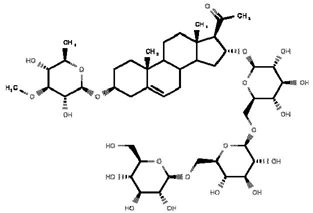 | *Myriopteron extensum* |
| | 979,076 | | | |
| **83** | C65H104O29 | no common name | 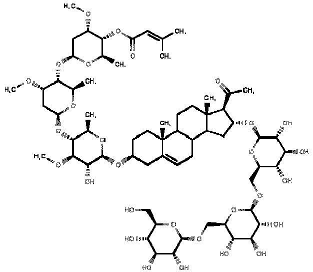 | *Myriopteron extensum* |
| | 1349,518 | | | |
| **84** | C65H104O29 | no common name | 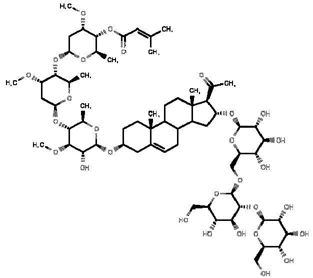 | *Myriopteron extensum* |
| | 1349,518 | | | |
| **85** | C51H84O25 | no common name | 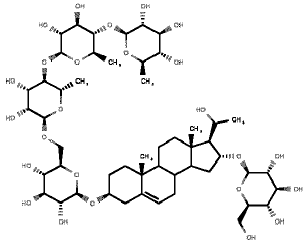 | *Myriopteron extensum* |
| | 1097,208 | | | |
| **86** | C45H74O21 | no common name | 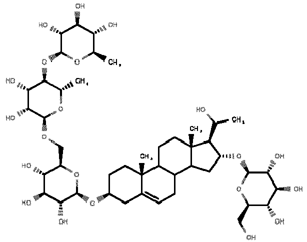 | *Myriopteron extensum* |
| | 951,066 | | | |
| **87** | C53H86O19S | no common name | 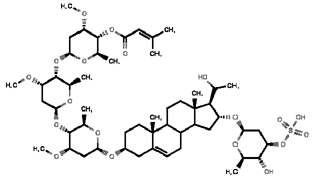 | *Myriopteron extensum* |
| | 1059,31 | | | |
| **88** | C27H42O3 | Diosgenin; (3beta,25R) Spirost-5-en-3-ol | 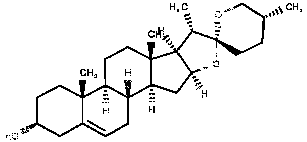 | *Dioscorea sp.* |
| | 414,63 | | | |
| | 512-04-9 | | | |
| **89** | C42H64O15 | Abrusoside C | 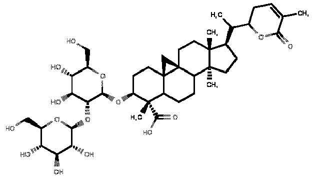 | *Abrus precatorius* |
| | 808,959 | | | |
| | 125002-99-5 | | | |
| **90** | C42H62O16 | Abrusoside E | 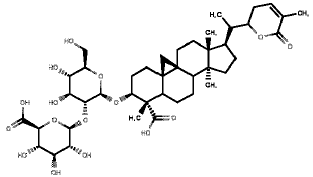 | *Abrus precatorius* |
| | 822,942 | | | |
| | 175448-27-8 | | | |
| **91** | C73H119O38 | Astersaponin C | 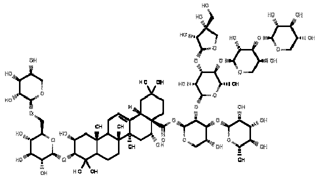 | *Aster tataricus* |
| | 1.603,71 | | | |
| | 125085-17-8 | | | |
| **92** | C42H64O16 | Periandrin III | 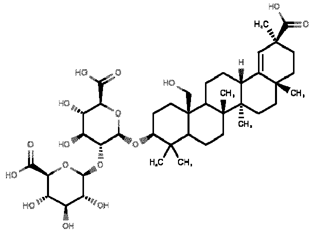 | *Periandra dulcis* |
| | 824,958 | | | |
| | 74256-70-5 | | | |
| **93** | C42H66O16 | Esculentoside S | 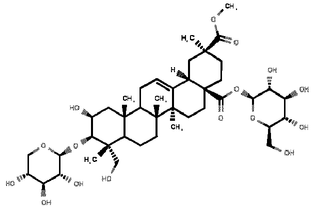 | *Saponaria sp.* |
| | 826,974 | | | |
| | 156031-83-3 | | | |
| **94** | C47H76O18 | Akebiasaponin D | 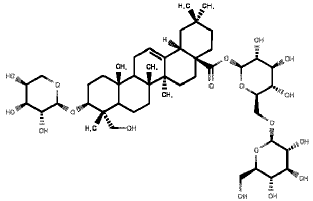 | *Dipsacus sp.* |
| | 929,107 | | | |
| | 39524-08-8 | | | |
| **95** | C63H102O29 | no common name | 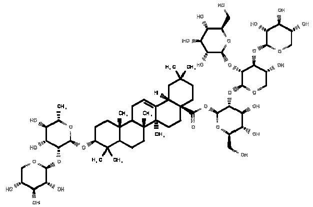 | *Albizia sp.* |
| | 1323,48 | | | |
| **96** | C42H72O14 | no common name | 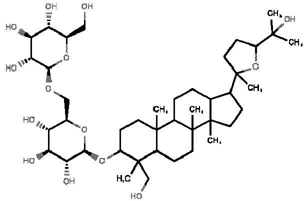 | *Gynostemma pentaphyllum* |
| | 801,024 | | | |
| **97** | C36H60O14 | no common name | 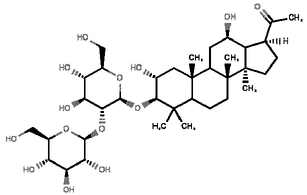 | *Gynostemma pentaphyllum* |
| | 716,862 | | | |
| **98** | C25H40O8 | Gaudichaudioside A | 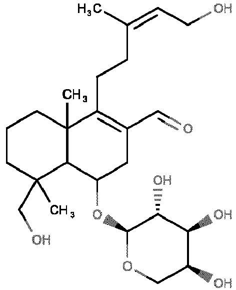 | *Baccharis gaudichaudiana* |
| | 468,587 | | | |
| | 138797-27-0 | | | |
| **99** | C25H44O8 | Gaudichaudioside D | 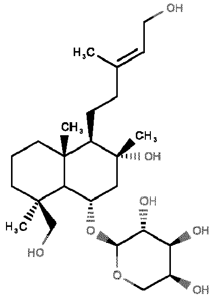 | *Baccharis gaudichaudiana* |
| | 472,619 | | | |
| | 138771-42-3 | | | |
| **100** | C25H42O8 | Gaudichaudioside B | 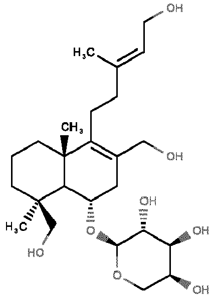 | *Baccharis gaudichaudiana* |
| | 470,603 | | | |
| | 138771-40-1 | | | |
| **101** | C31H48O14S | Phlomisoside II, 6'-sulfate | 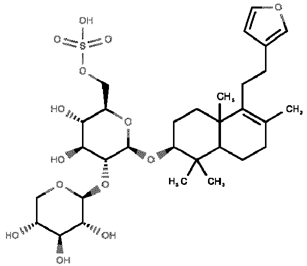 | *Phlomis betonicoides* |
| | 676,77 | | | |
| **102** | C31H48O14S | Phlomisoside II, 3'-sulfate | 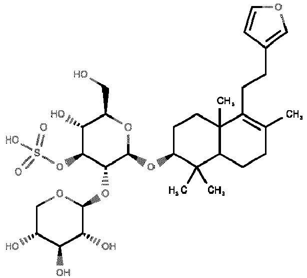 | *Phlomis betonicoides* |
| | 676,77 | | | |
| **103** | C32H50O12 | Phlomisoside II | 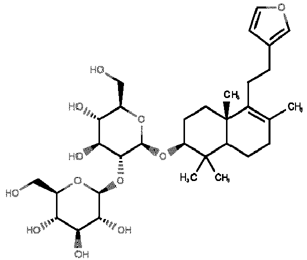 | *Phlomis betonicoides^{,}* |
| | 626,74 | | | |
| | 100414-78-6 | | | |
| **104** | C16H14O6 | Hesperetin | 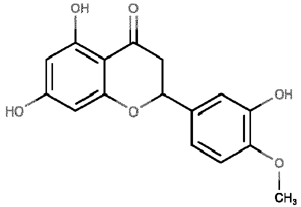 | *Citrus aurantium* |
| | 302,282 | | | |
| | 520-33-2 | | | |
| **105** | C16H14O7 | Padmatin | 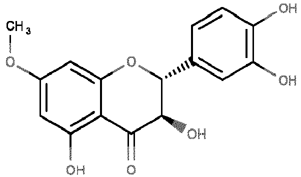 | *Artemisia erithmifolia* |
| | 318,281 | | | |
| | 80453-44-7 | | | |
| **106** | C20H18O10 | Kaempferol 3-a-L-arabinoside | 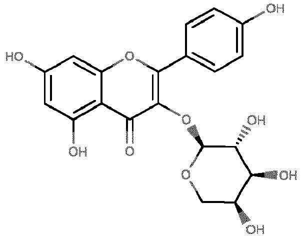 | *Euphorbia paralias. Rubus suavissimus* |
| | 418,354 | | | |
| | 99882-10-7 | | | |
| **107** | C15H12O6 | 3,3',4',7-Tetrahydroxyflavanone | 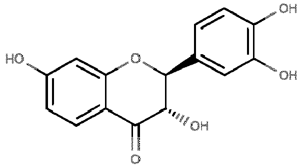 | *Perymenium grande* |
| | 288,255 | | | |
| | 20725-03-5 | | | |
| **108** | C21H22O11 | Neoisoastilbin | 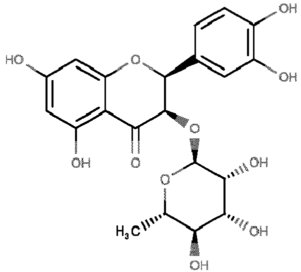 | *Engelhardtia crysolepis* |
| | 450,396 | | | |
| | 54141-72-9 | | | |
| **109** | C45H36O15 | Selligueain A | 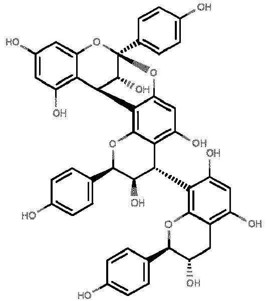 | *Polypodium vulgare* |
| | 816,768 | | | |
| | 152378-18-2 | | | |
| **110** | C21H24O9 | Gnetifolin E | 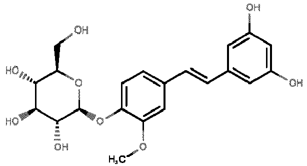 | *Gnetum sp.* |
| | 420,414 | | | |
| | 140671-07-4 | | | |
| **111** | C20H22O9 | Piceatannol 4'-O-β-D-glucopyranoside | 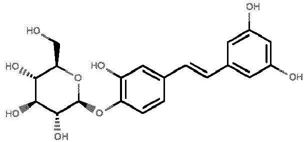 | *Bauhinia cardinalis* |
| | 406,387 | | | |
| | 116181-54-5 | | | |
| **112** | C16H14O6 | Thunberginol E | 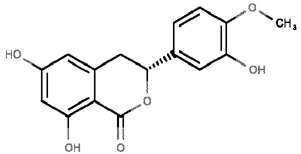 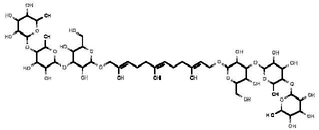 | *Hydrangea macrophylla* |
| | 302,282 | | | |
| | 147517-08-6 | | | |
| **113** | C51H86O28 | no common name | | *Sapindus saponaria* |
| | 1147,221 | | | |
| **114** | C28H57NO8 | no common name | 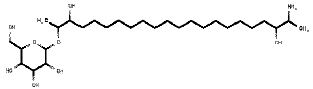 | *Fungi imperfecti* |
| | 535,763 | | | |
| **115** | C9H16O4 | Azelainsäure | 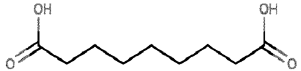 | *Solanum melongena* |
| | 188,223 | | | |
| | 123-99-9 | | | |
| **116** | C18H24O8 | Malaxinic acid | 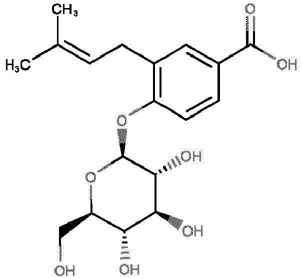 | *Prunus persica* |
| | 368,382 | | | |
| | 23179-40-0 | | | |
| **117** | C28H32O15 | no common name | 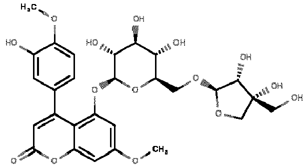 | *Hintonia latiflora* |
| | 608,549 | | | |
| **118** | C19H30O8 | 6,9-Dihydroxy-4,7-megastigmadien-3-one 9-O-β-D-Glucopyranoside 4-[(2,3-Dihydroxy-3-methyl-1-oxobutoxy)methyl]phenyl β-D-glucopyranoside | 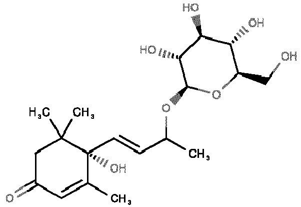 | *Citrus sinensis* |
| | 386,441 | | | |
| **119** | C18H26O10 | | 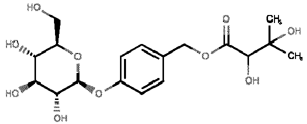 | *Cucurbita pepo* |
| | 402,396 | | | |
| | 321722-02-5 | | | |

2. A sweetener or sweetness enhancer composition comprising, consisting or essentially consisting of at least one of **Compounds 1 to 60** as defined in Claim 1.

3. The composition of Claim 2, further comprising at least one, preferably two, three or four **Compounds 61 to 119** as defined in Claim 1.

4. A method for
(i) sweetening a consumer product and/or
(ii) enhancing the sweetness of a consumer product of another sweetener, and/or
(iii) masking the unpleasant taste of another sweetener,
said sweetener preferably being a rebaudioside, and particularly being Rebaudioside D or Rebaudioside M, comprising the following steps:
(a) providing a consumer product or a sweetener different from compounds 1 to 60, and
(b) adding a composition comprising, consisting or essentially consisting of at least one, preferably two, three, four or more **Compounds 1 to 60** as defined in Claim 1.

5. The method of Claim 4, said compositions further comprising **Compounds 61 to 119** as defined in Claim 1.

6. A method for
(i) sweetening a consumer product and/or
(ii) enhancing the sweetness of a consumer product of another sweetener, and/or
(iii) masking the unpleasant taste of another sweetener,
said sweetener preferably being a rebaudioside, and particularly being Rebaudioside D or Rebaudioside M, comprising the following steps:
(a) providing a consumer product or a sweetener different from compounds 61 to 119, and
(b) adding a composition comprising, consisting or essentially consisting of at least one, preferably two, three, four or more **Compounds 61 to 119** as defined in Claim 1.

7. A method for
(i) sweetening a consumer product and/or
(ii) enhancing the sweetness of a consumer product of another sweetener, and/or
(iii) masking the unpleasant taste of another sweetener,
said sweetener preferably being a rebaudioside, and particularly being Rebaudioside D or Rebaudioside M, comprising the following steps:
(a) providing a consumer product or a sweetener different from compounds 1 to 15, and
(b) adding a composition comprising, consisting or essentially consisting of at least one, preferably two, three, four or more **Compounds 1 to 15** as defined in Claim 1.

8. The use of a composition comprising, consisting or essentially consisting of at least one, preferably two, three, four or more **Compounds 1 to 60** as defined in Claim 1 as sweeteners and/or sweetness enhancers and/or masking agents for the unpleasant taste of another sweetener, preferably as sweetener enhancers for rebaudiosides, in particularly for Rebaudioside D or Rebaudioside M.

9. The use of Claim 8, said compositions further comprising **Compounds 61 to 119** as defined in Claim 1.

10. The use of a composition comprising, consisting or essentially consisting of at least one, preferably two, three, four or more **Compounds 61 to 69** as defined in Claim 1 as sweeteners and/or sweetness enhancers and/or masking agents for the unpleasant taste of another sweetener, preferably as sweetener enhancers for rebaudiosides and more particularly for Rebaudioside D or Rebaudioside M.

11. The use of claim 9, the composition comprising, consisting or essentially consisting of at least one, preferably two, three, four or more **Compounds 1 to 15** as defined in Claim 1 as sweetness enhancers and/or masking agents for the unpleasant taste of another sweetener, preferably as sweetener enhancers for rebaudiosides, particularly for Rebaudioside M.

12. An extract comprising one or more **Compound 1 to 119** as defined in Claim 1 obtainable by aqueous and/or alcoholic extraction, said compounds and the respected plant or microorganisms from which they are obtained from, selected from the group consisting of:
| **Compound Reference** | ***Source*** |
|---|---|
| | |
| 1 | *Momordica grosvenori* |
| 2 | *Momordica grosvenori* |
| 3 | *Momordica grosvenori* |
| 4 | *Momordica grosvenori* |
| 5 | *Momordica grosvenori* |
| 6 | *Momordica grosvenori* |
| 7 | *Momordica grosvenori* |
| 8 | *Momordica grosvenori* |
| 9 | *Momordica grosvenori* |
| 10 | *Momordica grosvenori* |
| 11 | *Momordica grosvenori* |
| 12 | *Momordica grosvenori* |
| 13 | *Momordica grosvenori* |
| 14 | *Momordica grosvenori* |
| 15 | *Momordica grosvenori* |
| 16 | *Gynostemma pentaphyllum* |
| 17 | *Momordica grosvenori* |
| 18 | *Gynostemma pentaphyllum* |
| 19 | *Momordica grosvenori* |
| 20 | *Momordica grosvenori* |
| 21 | *Momordica grosvenori* |
| 22 | *Momordica grosvenori* |
| 23 | *Momordica grosvenori* |
| 24 | *Momordica grosvenori* |
| 25 | *Momordica grosvenori* |
| 26 | *Momordica grosvenori* |
| 27 | *Momordica grosvenori* |
| 28 | *Momordica grosvenori* |
| 29 | *Momordica grosvenori* |
| 30 | *Momordica grosvenori* |
| 31 | *Momordica grosvenori* |
| 32 | *Momordica grosvenori* |
| 33 | *Momordica grosvenori* |
| 34 | *Momordica grosvenori* |
| 35 | *Momordica grosvenori* |
| 36 | *Momordica grosvenori* |
| 37 | *Momordica grosvenori* |
| 38 | *Momordica grosvenori* |
| 39 | *Momordica grosvenori* |
| 40 | *Momordica grosvenori* |
| 41 | *Hypholoma fasciculare* |
| 42 | *Gynostemma pentaphyllum* |
| 43 | *Gynostemma pentaphyllum* |
| 44 | *Gynostemma pentaphyllum* |
| 45 | *Gynostemma pentaphyllum* |
| 46 | *Gynostemma pentaphyllum* |
| 47 | *Gynostemma pentaphyllum* |
| 48 | *Gynostemma pentaphyllum* |
| 49 | *Gynostemma pentaphyllum* |
| 50 | *Gynostemma pentaphyllum* |
| 51 | *Gynostemma pentaphyllum* |
| 52 | *Gynostemma pentaphyllum* |
| 53 | *Gynostemma pentaphyllum* |
| 54 | *Gynostemma pentaphyllum* |
| 55 | *Bryonia dioica* |
| 57 | *Bryonia dioica* |
| 58 | *Pterocarya paliurus* |
| 59 | *Pterocarya paliurus* |
| 60 | *Pterocarya paliurus* |
| 61 | *Abrus precatorius* |
| 62 | *Pterocarya paliurus* |
| 63 | *Pterocarya paliurus* |
| 64 | *Pterocarya paliurus* |
| 65 | *Pterocarya paliurus* |
| 66 | *Pterocarya paliurus* |
| 67 | *Pterocarya paliurus* |
| 68 | *Pterocarya paliurus* |
| 69 | *Pterocarya paliurus* |
| 70 | *Pterocarya paliurus* |
| 71 | *Pterocarya paliurus* |
| 72 | *Pterocarya paliurus* |
| 73 | *Pterocarya paliurus* |
| 74 | *Pterocarya paliurus* |
| 75 | *Pterocarya paliurus* |
| 76 | *Myriopteron extensum* |
| 77 | *Myriopteron extensum* |
| 78 | *Myriopteron extensum* |
| 79 | *Myriopteron extensum* |
| 80 | *Myriopteron extensum* |
| 81 | *Myriopteron extensum* |
| 82 | *Myriopteron extensum* |
| 83 | *Myriopteron extensum* |
| 84 | *Myriopteron extensum* |
| 85 | *Myriopteron extensum* |
| 86 | *Myriopteron extensum* |
| 87 | *Myriopteron extensum* |
| 88 | *Myriopteron extensum* |
| 89 | *Dioscorea sp.* |
| 90 | *Abrus precatorius* |
| 91 | *Aster tataricus* |
| 92 | *Abrus precatorius* |
| 93 | *Periandra dulcis* |
| 94 | *Saponaria sp.* |
| 95 | *Dipsacus sp.* |
| 96 | *Albizia sp.* |
| 97 | *Gynostemma pentaphyllum* |
| 98 | *Gynostemma pentaphyllum* |
| 99 | *Baccharis gaudichaudiana* |
| 100 | *Baccharis gaudichaudiana* |
| 101 | *Baccharis gaudichaudiana* |
| 102 | *Phlomis betonicoides* |
| 103 | *Phlomis betonicoides* |
| 104 | *Phlomis betonicoides* |
| 105 | *Citrus aurantium* |
| 106 | *Artemisia erithmifolia* |
| 107 | *Euphorbia paralias. Rubus suavissimus* |
| 108 | *Perymenium grande* |
| 109 | *Engelhardtia crysolepis* |
| 110 | *Polypodium vulgare* |
| 111 | *Gnetum sp.* |
| 112 | *Bauhinia cardinalis* |
| 113 | *Hydrangea macrophylla* |
| 114 | *Sapindus saponaria* |
| 115 | *Fungi imperfecti* |
| 116 | *Solanum melongena* |
| 117 | *Prunus persica* |
| 118 | *Hintonia latiflora* |
| 119 | *Citrus sinensis* |

13. A process for obtaining the extract of Claim 12 rich in **Compounds 1 to 119** as defined in Claim 1 encompassing or consisting of the following steps:
(a) providing a suspension of roots, leaves or aerial parts of a plant rich in the compound of interest in water, a C₁-C₄ aliphatic alcohol, acetone, ethyl acetate, methyl ethyl ketone, methyl-tert.-butyl ether, or their mixtures,
(b) subjecting said suspension to extraction using water, a C₁-C₄ aliphatic alcohol, acetone, ethyl acetate, methyl ethyl ketone, methyl-tert.-butyl ether, or their mixtures at temperatures of about 20 to about 50 °C, and optionally
(c) separating off the solvent.

14. A consumer product representing a food composition, an oral composition or a pharmaceutical composition comprising a working amount of the composition of Claim 1 or the extract of Claim 11.

15. The product of Claim 14 comprising the composition of Claim 1 or the extract of Claim 11 in an amount of 1 to about 2.000 ppm - calculated on the composition.
